# EUROPEAN PATENT SPECIFICATION

(11) **EP 3 630 074 B1**
(45) Date of publication and mention of the grant of the patent: **25.10.2023**
(21) Application number: 18727301.6
(22) Date of filing: 29.05.2018
(51) Int. Cl.: A61K 9/50, A61K 31/137, A61P 29/02

(54) **MULTIPARTICULATE ORAL DOSAGE FORM PROVIDING PROLONGED RELEASE OF TAPENTADOL**
MULTIPARTIKULÄRE ORALE DARREICHUNGSFORM MIT VERLÄNGERTER FREISETZUNG VON TAPENTADOL
FORME POSOLOGIQUE ORALE MULTIPARTICULAIRE À LIBÉRATION PROLONGÉE DE TAPENTADOL

(30) Priority: 29.05.2017 EP 17173240
(43) Date of publication of application: 08.04.2020
(73) Proprietor: Grünenthal GmbH, 52078 Aachen (DE)
(72) Inventor: SCHILLER, Marc, 52076 Aachen (DE); REINHOLD, Ulrich, 52076 Aachen (DE); BERTRAM, Ulrike, 52066 Aachen (DE); PRANGE, Wolfgang, 53501 Grafschaft (DE); PHILIPP, Anika-Anina, 52070 Aachen (DE); STRAUB, Stefanie, 61118 Bad Vilbel (DE); GRAVE, Annette, 79541 Lörrach (DE); POELLINGER, Norbert, 79379 Müllheim (DE)
(74) Representative: Kutzenberger Wolff & Partner
(86) International application number: PCT/EP2018/063991
(87) International publication number: WO 2018/219897

(56) References cited:
- EP-A1- 2 606 879
- WO-A1-2010/141505
- WO-A2-2011/124953
- US-A1- 2017 071 862
- US-B2- 8 895 063

## Description

The disclosure relates to an oral pharmaceutical dosage form comprising a plurality of coated particles, wherein said coated particles comprise a core which comprises a Tapentadol component and which is coated with a controlled release coating material (controlled release coat), wherein the controlled release coating material comprises a lubricant component and a polymer component, wherein the polymer component comprises one or more cellulose ethers, and wherein the pharmaceutical dosage form provides controlled release of the Tapentadol component.
The invention relates to an oral pharmaceutical dosage form comprising a plurality of coated particles, wherein said coated particles comprise a core which comprises a Tapentadol component, wherein under *in vitro* conditions the pharmaceutical dosage form provides controlled release of the Tapentadol component, and wherein the core is coated with a controlled release coating material (controlled release coat), wherein the controlled release coating material comprises a lubricant component and a polymer component, wherein the lubricant component comprises or essentially consists of magnesium stearate, wherein the polymer component comprises one or more cellulose ethers, and wherein the relative weight ratio of the polymer component to the lubricant component is within the range of from 5.0:1.0 to 2.0:1.0.

Tapentadol (brand names: Nucynta^{®} and Palexia^{®}) is a centrally acting opioid analgesic of the benzenoid class with a dual mode of action as an agonist of the µ-opioid receptor and as a norepinephrine reuptake inhibitor (NRI).

Nucynta^{®} or Palexia^{®} extended release (tapentadol) is indicated for the management of: (i) pain severe enough to require daily, around-the-clock, long-term opioid treatment and for which alternative treatment options are inadequate; (ii) neuropathic pain associated with diabetic peripheral neuropathy (DPN) in adults severe enough to require daily, around-the-clock, long-term opioid treatment and for which alternative treatment options are inadequate.

There is an increasing number of patients, especially pediatric patients and geriatric patients, who have difficulties in swallowing monolithic oral dosage forms. In addition, multiparticulate dosage forms have advantages for the use in patients with gastric or duodenal tubes.

Furthermore, the release kinetics of the pharmacologically active ingredients is an important factor. It is well known that depending on how a pharmacologically active ingredient is formulated into a dosage form its release pattern can be modified.

On the one hand, formulations providing immediate release upon oral administration have the advantage that they lead to a fast release of the pharmacologically active ingredient in the gastrointestinal tract. As a result, a comparatively high dose of the pharmacologically active ingredient is quickly absorbed leading to high plasma levels within a short period of time and resulting in a rapid onset of medicinal action, i.e. medicinal action begins shortly after administration. At the same time, however, a rapid reduction in the medicinal action is observed, because metabolization and/or excretion of the pharmacologically active ingredient cause a decrease of plasma levels. For that reason, formulations providing immediate release of pharmacologically active ingredients typically need to be administered frequently, e.g. six times per day. This may cause comparatively high peak plasma pharmacologically active ingredient concentrations and high fluctuations between peak and trough plasma pharmacologically active ingredient concentrations which in turn may deteriorate tolerability.

Controlled release (e.g. delayed release, prolonged release, sustained release, extended release and the like) may be based upon various concepts such as coating the pharmaceutical dosage form with a controlled release membrane, embedding the pharmacologically active ingredient in a matrix, binding the pharmacologically active ingredient to an ion-exchange resin, forming a complex of the pharmacologically active ingredient, and the like. In this context it can be referred to, e.g., W. A. Ritschel, Die Tablette, 2. Auflage, Editio Cantor Verlag Aulendorf, 2002. In comparison to formulations providing immediate release, formulations providing prolonged release upon oral administration have the advantage that they need to be administered less frequently, typically once daily or twice daily. This can reduce peak plasma pharmacologically active ingredient concentrations and fluctuations between peak and trough plasma pharmacologically active ingredient concentrations which in turn may improve tolerability.

However, especially patients starting their treatment with controlled release formulations often desire a rapid onset of medicinal action. Therefore, a need exists to develop oral formulations which provide a quick medicinal action while at the same time having the benefits of controlled or modified release formulations.

Further, the potential impact of concomitant intake of ethanol on the in vivo release of drugs from modified release oral formulations (dose dumping) has recently become an increasing concern. Controlled or modified release formulations typically contain a higher amount of the pharmacologically active ingredient relative to its immediate release counterpart. If the controlled release portion of the formulation is easily defeated, the end result is a potential increase in exposure to the active drug and possible safety concerns. In order to improve safety and circumvent intentional tampering (e.g. dissolving a controlled release pharmaceutical dosage form in ethanol to extract the drug), a reduction in the dissolution of the modified release fractions of such formulations, in ethanol, may be of benefit. Accordingly, the need exists to develop new formulations having reduced potential for dose dumping in alcohol.

WO 2007/093642 aims at minimizing the risks of release of the dose associated with the concurrent consumption of alcohol and certain pharmaceutical or dietary forms with modified release. The reference concerns an oral form comprising reservoir-type microparticles, with modified release of at least one active principle. The oral form is resistant to the immediate release of the dose of active principle in the presence of alcohol. In particular, the oral form is characterized in that the releasing time of 50% of the active principle, in an alcohol-containing solution is not reduced by more than 3 times compared to the releasing time of 50% of the active principle in an alcohol-free aqueous medium.

WO 2008/033523 relates to a pharmaceutical composition that may include a granulate which may include at least one active pharmaceutical ingredient susceptible to abuse by an individual mixed with at least two materials, a first material that is substantially water insoluble and at least partially alcohol soluble and a second material that is substantially alcohol insoluble and at least partially water soluble, wherein the active pharmaceutical ingredient and the two materials are granulated in the presence of water and alcohol. The composition may also include a coating on the granulate exhibiting crush resistance which may have a material that is deposited on the granulate using an alcohol based solvent. The composition further comprises a second particle comprising a fat/wax.

WO 2010/037854 relates to an oral pharmaceutical form containing microgranules for the sustained release of at least one active principle, including a neutral carrier that is insoluble in water or in an alcoholic solution, or a neutral carrier rendered insoluble in water or an alcoholic solution, comprising at least one first mounting layer containing at least one active principle and optionally a pharmaceutically acceptable binding agent, wherein the assembly comprises at least one coating containing at least one hydrophobic polymer. WO 2010/141505 discloses dosage forms comprising at least one opioid agonist prone to abuse and at least one opioid antagonist, effective to enhance the therapeutic potency of the opioid agonists and reduce their abuse or providing effective therapeutic relief for at least 12 hours.

WO 2011/124953 discloses a once daily controlled release pharmaceutical compositions comprising Tapentadol, wherein preferably the mean Tmax of Tapentadol is reached after 10 hours of administration of the composition. The composition comprises Tapentadol, such that it maintains serum concentration of Tapentadol of at least about 20 ng/ml for at least about 17 hours after oral administration of the composition. According to one embodiment the controlled release pharmaceutical composition comprises Tapentadol, which is gastroretentive.

WO 2011/141241 relates to an oral pharmaceutical composition which is resistant to immediate discharge of the dose of active ingredient due to alcohol and which enables a single daily intake. EP 2 606 879 relates to a sustained-release pharmaceutical formulation, in particular a multiple unit pellet tablet (MUT) formulation for oral administration comprising a plurality of sustained-release pellets.

WO 2012/166474 is directed to a solid dose form comprising a film coating composition encapsulating a core, wherein: (i) the core comprises an active ingredient comprising at least one of a pharmaceutical, veterinary, or nutraceutical active ingredient; (ii) the film coating composition comprises ethylcellulose and guar gum; (iii) the dose form provides controlled release of the active ingredient; (iv) the guar gum is present in an amount greater than 5 wt% based on the weight of the guar gum and ethylcellulose; and (v) the dose form is ethanol resistant.

WO 2014/032741 relates to a pharmaceutical or nutraceutical composition with a core, an inner layer, and an outercoating layer, wherein a pharmaceutical or a nutraceutical active ingredient is contained in the core, at least 30% by weight of a salt of alginic acid is contained in the inner layer, and at least 30% by weight of a polymer or copolymer with anionic side groups is contained in the outer coating layer. US 8 895 063 relates to an oral solid dosage form containing one or several active principle(s) having analgesic properties, the composition of said dosage form being such that it prevents the misuse of said dosage form through the liquid extraction of the active principle(s) contained therein, using commonly available solvents.

N. Jedinger et al., Eur J Pharm Biopharm 87 (2014) 217-226 reviews the design of controlled-release formulations resistant to alcohol-induced dose dumping. US 2017/071862 relates to a pharmaceutical dosage form which is particularly useful for the prevention of an overdose of the pharmacologically active ingredient contained therein after accidental or intentional simultaneous administration of a plurality of the dosage forms containing an overall supratherapeutic dose of the pharmacologically active ingredient.

The dosage forms of the prior art are not satisfactory in every respect and there is a demand for alternative or improved dosage forms.

It is an object of the invention to provide pharmaceutical dosage forms that have advantages compared to the prior art, especially pharmaceutical dosage forms comprising Tapentadol. The pharmaceutical dosage forms should provide controlled release of Tapentadol and should be bioequivalent to the commercial forms of pharmaceutical dosage forms comprising Tapentadol and providing prolonged release thereof (Palexia^{®}, Nucynta^{®}).

A first aspect of the invention relates to an oral pharmaceutical dosage form comprising a plurality of coated particles, wherein said coated particles comprise a core which comprises a Tapentadol component and which is coated with a controlled release coating material, wherein the controlled release coating material comprises a lubricant component and a polymer component, wherein the lubricant component comprises or essentially consists of magnesium stearate, wherein the polymer component comprises one or more cellulose ethers, and wherein under *in vitro* conditions the pharmaceutical dosage form provides controlled release of the Tapentadol component. Preferably, the weight content of the controlled release coating material, relative to the total weight of the coated particles, is less than 50 wt.-%.

It has been surprisingly found that the lubricant component, especially magnesium stearate, has a retardant effect when it is employed in such comparatively high concentrations in a controlled release coating material which additionally comprises a polymer component, especially ethylcellulose.

The coated particles that are contained in the dosage form according to the invention can be easily taken by a subject in need thereof, either in form of the dosage form as such (e.g. whole capsule) or in form of the coated particles (e.g. particles removed from a sprinkle capsule).

Further, it has been surprisingly found that a subcoat between the core and the controlled release coating material can be omitted. Subcoats are usually necessary in order to avoid migration of the active ingredient from the inside to the outside thereby altering the release characteristics and sometimes also the stability upon storage.

Still further, it has been surprisingly found that by mixing the coated particles according to the invention with particles providing immediate release of a Tapentadol component, bimodal or multimodal release kinetics can be achieved, thereby e.g. achieving a rapid onset of medicinal action.

Yet further, it has been surprisingly found that by mixing the coated particles according to the invention with particles providing release of pharmacologically active ingredients other than Tapentadol component, combination formulations can be easily provided at different dosage strength. Thus, the invention can be regarded as part of a building block or intermediate for the simplified preparation of a large variety of different dosage forms providing different release kinetics, optionally of different pharmacologically active ingredients including a Tapentadol component.

Furthermore, it has been found that the *in vitro* release profile of the dosage form according to the invention has sigmoid shape and in this regard substantially differs from the commercial tablet providing prolonged release of a Tapentadol component at the same dose with a root z shape of the *in vitro* release profile. Nonetheless, in spite of the substantially different *in vitro* release profiles, both dosage forms are unexpectedly bioequivalent to one another (confidence interval 80 to 125%), especially with regard to Cₘₐₓ. Thus, any pharmacokinetic and pharmacodynamic advantages that can be achieved by said commercial tablet can also analogously be achieved with the dosage form according to the invention.

Further, it has been surprisingly found that the *in vitro* release profile of the dosage form according to the invention is very robust according to variations of release kinetics still maintaining bioequivalence with respect to said commercial tablet. This is likely also supported by *in vitro in vivo* correlation (IVIVC), i.e. a predictive mathematical model describing the relationship between an *in vitro* property of a dosage form and an *in vivo* response.

Moreover, it has been surprisingly found that the *in vitro* release profile of the dosage form according to the invention can be altered by varying the particle size of the lubricant component. It has been surprisingly found that the *in vitro* release profile can be accelerated when the average particle size of the lubricant component is reduced. Thus, the *in vitro* release profile of the dosage form according to the invention can be tailored to the desired needs not only by changing the amount of the relative weight ratio of the lubricant component and the polymer component to one another and the overall thickness of the controlled release coating, but additionally by the particle size of the lubricant component within the controlled release coating material.

The pharmaceutical dosage form according to the invention is an oral dosage form, i.e. devoted for oral administration.

The pharmaceutical dosage form according to the invention comprises a plurality of coated particles.

The coated particles comprise each a core. The core comprises, comprises essentially, or consists of the Tapentadol component.

The core may comprise or may contain a neutral carrier pellet, for instance a sugar sphere or nonpareilles, on top of which the Tapentadol component may be bound in a binder, such as lactose, celluloses, like micro crystalline cellulose (MCC), or polyvinylpyrrolidon (PVP). In this case the Tapentadol component may be bound or placed localized at the surface of the core (as a part of the core). The binding of the Tapentadol component at the surface of the core in such a binding layer has usually no effect or influence in the sense of a release control function.

The core may alternatively comprise a pellet in the form of a polymeric matrix in which the Tapentadol component is bound. The core may comprise an uncoated pellet or granule consisting of a crystallized Tapentadol component.

In a preferred embodiment, the core can be regarded as a starter pellet (e.g. a nonpareil) as such not comprising a pharmacologically active ingredient. Starter pellets are commercially available and preferably comprise an inert carrier such as microcrystalline cellulose. Thus, according to this embodiment, the Tapentadol component is contained in a drug layer with which the starter pellet is coated (drug coat). Starter pellet and drug coat form the core that comprises the Tapentadol component.

In another preferred embodiment, the core does not comprise a starter pellet but preferably a homogeneous mixture of the Tapentadol component with one or more excipients (e.g. fillers, binders, and the like).

According to this embodiment, besides the Tapentadol component, the core preferably comprises one or more binders, fillers, and the like, preferably one or more binders. Preferred binders include but are not limited to cellulose and cellulose derivatives, such as microcrystalline cellulose or cellulose ethers; starches such as maize starch, rice starch, potato starch and wheat starch, modified starches (e.g. pregelatinized starches); gelatin, tragacanth, polyvinylpyrrolidone (e.g. Povidone K12-K30); and the like as well as the mixtures thereof.

Preferred cellulose ethers include but are not limited to methylcellulose, ethylcellulose, propylcellulose, hydroxypropylcellulose, hydroxypropylmethylcellulose and carboxymethylcellulose as well as the salts thereof. Cellulose and hydroxypropylmethylcellulose are particularly preferred, whereas the latter is preferably low substituted in accordance with Ph. Eur.

In a preferred embodiment, the core comprises a homogenous mixture of the Tapentadol component, microcrystalline cellulose and hydroxypropylmethylcellulose, preferably low substituted in accordance with Ph. Eur. Preferably, the relative weight ratio of microcrystalline cellulose and hydroxypropylmethylcellulose is within the range of from 3:1 to 1:3, more preferably 2:1 to 1:2 and in particular 1.5:1 to 1:1.5.

Thus, according to this embodiment, the Tapentadol component is preferably homogeneously distributed in the core which does not comprise a starter pellet. This embodiment is particularly preferred when the coated particles are to form an oral dosage form comprising a comparatively high dose of the Tapentadol component (e.g. 150 mg and more). At such high doses, starter pellets would significantly increase the overall volume of the coated particles such that they could hardly be filled into a capsule that is small enough to be easily swallowed. Thus, under these circumstances, it can be preferred to avoid starter pellets but to provide cores containing the Tapentadol component at comparatively high content.

Alternatively, it is also possible to provide a corresponding amount of sprinkled pellets. Certain drug products that contain beads within a capsule indicate in the labeling that the capsule can be broken and the internal beads can be sprinkled on soft foods and swallowed without chewing as an alternative administration technique.

In preferred embodiments of the invention, the core
(i) may be regarded as a starter pellet with an inert carrier (e.g. nonpareil) comprising a drug coat which preferably contains essentially the total amount of the Tapentadol component;
(ii) comprises a homogeneous mixture of preferably essentially the total amount of the Tapentadol component and one or more excipients; or
(iii) comprises a homogeneous mixture of a portion of the total amount of the Tapentadol component and one or more excipients as well as a drug coat which contains the remainder of the total amount of the Tapentadol component.

The core of the plurality of coated particles comprises a Tapentadol component. For the purpose of the specification, a Tapentadol component encompasses Tapentadol free base or a physiologically acceptable salt thereof, in solid form, e.g. in crystalline form, in form of any polymorph, solvate, co-crystal, aggregate and the like. Preferably, the Tapentadol component is Tapentadol hydrochloride.

Unless expressly stated otherwise, all amounts and percentages of the Tapentadol component are expressed as equivalent weights and percentages, respectively, of Tapentadol free base.

The core of the plurality of coated particles is coated with a controlled release coating material.

Thus, according to a preferred embodiment, when the core of the particles comprises a homogenous mixture of the Tapentadol component and one or more excipients, the coating of controlled release coating material (controlled release coat) is preferably the innermost coating of the core. Alternatively, according to another preferred embodiment, when the core of the particles already comprises a drug coat that comprises the Tapentadol component, it additionally comprises another coating, namely the coating of controlled release coating material (controlled release coat).

The controlled release coating material serves the purpose of controlling release of the Tapentadol component from the coated particles. Said controlled release coat of the controlled release coating material may form the outer surface of the particles or may be further overcoated with one or more layers of different or identical coating materials, e.g. in order to render the particles resistant against ethanolic dose dumping.

The pharmaceutical dosage form according to the invention provides controlled release of the Tapentadol component. For the purpose of the specification, controlled release means not immediate release. Controlled release includes delayed release (extended release), staggered release (repeat action release), sustained release (prolonged release) and evenly sustained release (sustained release). For purposes of describing "delayed release" preferably a sustained release of physiologically active substance for a defined, finite period of time (lag time), the release is unhindered by the end. "Staggered release" means the initial release of a first subset of the physiologically active substance followed by at least one other subset, which is released subsequently. "Prolonged release" means a release with a reduced release rate, to obtain a therapeutic effect upright, to reduce toxic effects or for some other therapeutic purpose. "Sustained release" means a continuous release over a relatively long period of time to reduce the frequency of administration. For further details, for example, can be made to K.H. Bauer, Textbook of Pharmaceutical Technology, 6th edition, WVG Stuttgart, 1999; and the European Pharmacopoeia.

Preferably, the content of the Tapentadol component in the controlled release coating material is not more than 5.0 wt.-%, more preferably not more than 4.0 wt.-%, still more preferably not more than 3.0 wt.-%, yet more preferably not more than 2.0 wt.-%, even more preferably not more than 1.5 wt.-%, most preferably not more than 1.0 wt.-%, and in particular not more than 0.5 wt.-%, relative to the total weight of the controlled release coating material. Preferably, the controlled release coating material comprises essentially no Tapentadol component.

Preferably, the controlled release coating material forms a layer (controlled release coat) having an average thickness of at least 1.0 µm, more preferably at least 2.0 µm, still more preferably at least 3.0 µm, yet more preferably at least 4.0 µm, even more preferably at least 5.0 µm, most preferably at least 6.0 µm, and in particular at least 7.0 µm.

Preferably, the controlled release coating material forms a layer (controlled release coat) having an average thickness of not more than 20 µm, more preferably not more than 19 µm, still more preferably not more than 18 µm, yet more preferably not more than 17 µm, even more preferably not more than 16 µm, most preferably not more than 15 µm, and in particular not more than 14 µm.

Preferably, the controlled release coating material forms a layer (controlled release coat) having an average thickness within the range of 11.0±9.0 µm, more preferably 11.0±8.0 µm, still more preferably 11.0±7.0 µm, yet more preferably 11.0±6.0 µm, even more preferably 11.0±5.0 µm, most preferably 11.0±4.0 µm, and in particular 11.0±3.0 µm.

The thickness of this layer can be easily determined, e.g. based on electron micrographs.

Preferably, the controlled release coating material i.e. the controlled release coat, forms the outer surface of the coated particles. Typically, the total surface of the particles is coated with the controlled release coating material such that the particles are completely covered by and encapsulated with the controlled release coating material, respectively.

In a preferred embodiment, the controlled release coating material is coated with one or more layers of one or more different coating materials, e.g. tamper-resistant coating materials in order to render the particles resistant against ethanolic dose dumping (tamper resistant coat). Such tamper resistant coat may in turn comprise one or more layers of different or identical coating materials.

In another preferred embodiment, the controlled release coating material forms the outer coating (outer surface) of the coated particles, i.e. is exposed to the environment.

Preferably, the weight content of the controlled release coating material, relative to the total weight of the coated particles, is not more than 45.5 wt.-% or not more than 49 wt.-%, more preferably not more than 47.5 wt.-% or not more than 45 wt.-%, still more preferably not more than 42.5 wt.-% or not more than 40 wt.-%, yet more preferably not more than 37.5 wt.-% or not more than 35 wt.-%, even more preferably not more than 32 wt.-% or not more than 31 wt.-%, most preferably not more than 30 wt.-% or not more than 29 wt.-%, and in particular not more than 28 wt.-% or not more than 27 wt.-%.

Preferably, the weight content of the controlled release coating material, relative to the total weight of the coated particles, is not more than 26 wt.-% or not more than 25 wt.-%, more preferably not more than 24 wt.-% or not more than 23 wt.-%, still more preferably not more than 22 wt.-% or not more than 21 wt.-%, yet more preferably not more than 20 wt.-% or not more than 19 wt.-%, even more preferably not more than 18 wt.-% or not more than 17 wt.-%, most preferably not more than 16 wt.-% or not more than 15 wt.-%, and in particular not more than 14 wt.-% or not more than 13 wt.-%.

Preferably, the weight content of the controlled release coating material, relative to the total weight of the coated particles, is within the range of from 5.0 wt.-% to 23 wt.-%, or from 7.0 wt.-% to 22 wt.-%, or from 8.0 wt.-% to 21 wt.-%, or from 5.0 wt.-% to 21 wt.-%, more preferably from 7.0 wt.-% to 19 wt.-%.

In preferred embodiments, the weight content of the controlled release coating material, relative to the total weight of the coated particles, is within the range of 9.0±8.0 wt.-%, or 9.0±7.0 wt.-%, or 9.0±6.0 wt.-%, or 9.0±5.0 wt.-%, or 9.0±4.0 wt.-%, more preferably 9.0±3.5 wt.-%, still more preferably 9.0±3.0 wt.-%, yet more preferably 9.0±2.5 wt.-%, even more preferably 9.0±2.0 wt.-%, most preferably 9.0±1.5 wt.-%, and in particular 9.0±1.0 wt.-% or 9.0±0.5 wt. %.

In preferred embodiments, the weight content of the controlled release coating material, relative to the total weight of the coated particles, is within the range of 11.0±8.0 wt.-%, or 11.0±7.0 wt.-%, or 11.0±6.0 wt.-%, or 11.0±5.0 wt. %, or 11.0±4.0 wt. %, more preferably 11.0±3.5 wt.-%, still more preferably 11.0±3.0 wt.-%, yet more preferably 11.0±2.5 wt.-%, even more preferably 11.0±2.0 wt.-%, most preferably 11.0±1.5 wt.-%, and in particular 11.0±1.0 wt.-% or 11.0±0.5 wt. %.

In preferred embodiments, the weight content of the controlled release coating material, relative to the total weight of the coated particles, is within the range of 13.0±8.0 wt.-%, or 13.0±7.0 wt.-%, or 13.0±6.0 wt.-%, or 13.0±5.0 wt.-%, or 13.0±4.0 wt.-%, more preferably 13.0±3.5 wt.-%, still more preferably 13.0±3.0 wt.-%, yet more preferably 13.0±2.5 wt.-%, even more preferably 13.0±2.0 wt.-%, most preferably 13.0±1.5 wt.-%, and in particular 13.0±1.0 wt.-% or 13.0±0.5 wt.-%.

In preferred embodiments, the weight content of the controlled release coating material, relative to the total weight of the coated particles, is within the range of 15.0±8.0 wt.-%, or 15.0±7.0 wt.-%, or 15.0±6.0 wt.-%, or 15.0±5.0 wt.-%, or 15.0±4.0 wt.-%, more preferably 15.0±3.5 wt.-%, still more preferably 15.0±3.0 wt.-%, yet more preferably 15.0±2.5 wt.-%, even more preferably 15.0±2.0 wt.-%, most preferably 15.0±1.5 wt.-%, and in particular 15.0±1.0 wt.-% or 15.0±0.5 wt. %.

In preferred embodiments, the weight content of the controlled release coating material, relative to the total weight of the coated particles, is within the range of 17.0±8.0 wt.-%, or 17.0±7.0 wt.-%, or 17.0±6.0 wt.-%, or 17.0±5.0 wt. %, or 17.0±4.0 wt. %, more preferably 17.0±3.5 wt.-%, still more preferably 17.0±3.0 wt.-%, yet more preferably 17.0±2.5 wt.-%, even more preferably 17.0±2.0 wt.-%, most preferably 17.0±1.5 wt.-%, and in particular 17.0±1.0 wt.-% or 17.0±0.5 wt. %.

In preferred embodiments, the weight content of the controlled release coating material, relative to the total weight of the coated particles, is within the range of 19.0±8.0 wt.-%, or 19.0±7.0 wt.-%, or 19.0±6.0 wt.-%, or 19.0±5.0 wt.-%, or 19.0±4.0 wt.-%, more preferably 19.0±3.5 wt.-%, still more preferably 19.0±3.0 wt.-%, yet more preferably 19.0±2.5 wt.-%, even more preferably 19.0±2.0 wt.-%, most preferably 19.0±1.5 wt.-%, and in particular 19.0±1.0 wt.-% or 19.0±0.5 wt. %.

In preferred embodiments, the weight content of the controlled release coating material, relative to the total weight of the coated particles, is within the range of 22.5±8.0 wt.-%, or 22.5±7.0 wt.-%, or 22.5±6.0 wt.-%, or 22.5±5.0 wt.-%, or 22.5±4.0 wt.-%, more preferably 22.5±3.5 wt.-%, still more preferably 22.5±3.0 wt.-%, yet more preferably 22.5±2.5 wt.-%, even more preferably 22.5±2.0 wt.-%, most preferably 22.5±1.5 wt.-%, and in particular 22.5±1.0 wt.-% or 22.5±0.5 wt. %.

In preferred embodiments, the weight content of the controlled release coating material, relative to the total weight of the coated particles, is within the range of 25.0±8.0 wt.-%, or 25.0±7.0 wt.-%, or 25.0±6.0 wt.-%, or 25.0±5.0 wt.-%, or 25.0±4.0 wt.-%, more preferably 25.0±3.5 wt.-%, still more preferably 25.0±3.0 wt.-%, yet more preferably 25.0±2.5 wt.-%, even more preferably 25.0±2.0 wt.-%, most preferably 25.0±1.5 wt.-%, and in particular 25.0±1.0 wt.-% or 25.0±0.5 wt. %.

According to the disclosure, the controlled release coating material comprises
(i) a lubricant component and a polymer component.

According to the invention, the controlled release coating material comprises a lubricant component, which comprises or essentially consists of magnesium stearate, and polymer component (embodiment (i)).

Preferably, the total content of the lubricant component and the polymer component is at least 80 wt.-%, more preferably at least 85 wt.-%, still more preferably at least 90 wt.-%, yet more preferably at least 92.5 wt.-%, even more preferably at least 95 wt.-%, most preferably at least 97.5 wt.-%, and in particular at least 98.5 wt.-%, relative to the total weight of the controlled release coating material.

Preferably, the controlled release coating material essentially consists of the lubricant component and the polymer component.

According to the invention, the relative weight ratio of the polymer component to the lubricant component is within the range of from 5.0:1.0 to 2.0:1.0, preferably from 5.0:1.0 to 2.1:1.0, more preferably from 4.7:1.0 to 2.2:1.0, yet more preferably from 4.4:1.0 to 2.4:1.0, even more preferably from 4.1:1.0 to 2.6:1.0, most preferably from 3.8:1.0 to 2.8:1.0 and in particular from 3.5:1.0 to 3.0:1.0.

In a preferred embodiment, the polymer component comprises or essentially consists of a cellulose ether selected from the group consisting of ethylcellulose, hydroxyethylcellulose, propylcellulose, hydroxypropylcellulose, hydroxypropyl methylcellulose, and mixtures thereof.

Preferably, the cellulose ether is ethylcellulose. The ethylcellulose (i.e. partly O-ethylated cellulose) is in accordance with Ph. Eur. and thus has a content of 44.0 wt.-% to 51.0 wt.-% of ethoxy (-OC₂H₅) groups (dried substance). In preferred embodiments, said content of ethoxy groups is within the range of 45.0±1.0 wt.-%, or 46±2.0 wt.-%, or 46±1.0 wt.-%, or 47±2.0 wt.-%, or 47±1.0 wt.-%, or 48±2.0 wt.-%, or 48±1.0 wt.-%, or 49±2.0 wt.-%, or 49±1.0 wt.-%, or 50±1.0 wt.-%.

Preferably, the weight content of the polymer component, relative to the total weight of the controlled release coating material, is at least 67 wt.-%, more preferably at least 68 wt.-%, still more preferably at least 69 wt.-%, yet more preferably at least 70 wt.-%, even more preferably at least 71 wt.-%, most preferably at least 72 wt.-%, and in particular at least 73 wt.-%.

Preferably, the weight content of the polymer component, relative to the total weight of the controlled release coating material, is not more than 87 wt.-%, more preferably not more than 86 wt.-%, still more preferably not more than 85 wt.-%, yet more preferably not more than 84 wt.-%, even more preferably not more than 83 wt.-%, most preferably not more than 82 wt.-%, and in particular not more than 81 wt. %.

Preferably, the weight content of the polymer component, relative to the total weight of the controlled release coating material, is within the range of 77.0±9.0 wt.-%, more preferably 77.0±8.0 wt.-%, still more preferably 77.0±7.0 wt.-%, yet more preferably 77.0±6.0 wt.-%, even more preferably 77.0±5.0 wt.-%, most preferably 77.0±4.0 wt.-%, and in particular 77.0±3.0 wt.-%.

Preferably, the weight content of the polymer component, relative to the total weight of the coated particles, is at least 5.0 wt.-%, more preferably at least 5.5 wt.-%, still more preferably at least 6.0 wt.-%, yet more preferably at least 6.5 wt.-%, even more preferably at least 7.0 wt.-%, most preferably at least 7.5 wt.-%, and in particular at least 8.0 wt.-%.

Preferably, the weight content of the polymer component, relative to the total weight of the coated particles, is not more than 20 wt.-%, or not more than 19 wt.-%, or not more than 18 wt.-%, or not more than 17 wt.-%, or not more than 16 wt.-%, or not more than 15 wt.-%, more preferably not more than 14.5 wt.-%, still more preferably not more than 14 wt.-%, yet more preferably not more than 13.5 wt.-%, even more preferably not more than 13 wt.-%, most preferably not more than 12.5 wt.-%, and in particular not more than 12 wt. %.

In a preferred embodiment, the weight content of the polymer component, relative to the total weight of the coated particles, is within the range of 10.0±9.0 wt.-%, more preferably 10.0±8.0 wt.-%, still more preferably 10.0±7.0 wt.-%, yet more preferably 10.0±6.0 wt.-%, even more preferably 10.0±5.0 wt.-%, most preferably 10.0±4.0 wt.-%, and in particular 10.0±3.0 wt.-%.

In another preferred embodiment, the weight content of the polymer component, relative to the total weight of the coated particles, is within the range of 15.0±14.0 wt.-%, or 15.0±13.0 wt.-%, or 15.0±12.0 wt.-%, or 15.0±11.0 wt. %, or 15.0±10.0 wt.-%, or 15.0±9.0 wt.-%, more preferably 15.0±8.0 wt.-%, still more preferably 15.0±7.0 wt.-%, yet more preferably 15.0±6.0 wt.-%, even more preferably 15.0±5.0 wt.-%, most preferably 15.0±4.0 wt.-%, and in particular 15.0±3.0 wt.-%.

In a preferred embodiment, the lubricant component, which comprises or essentially consists of magnesium stearate, additionally comprises a fatty acid, a further metallic salt of a fatty acid, a fatty acid ester, an inorganic material, a polymeric lubricant, or a mixture thereof.

In preferred embodiments,
- the fatty acid is selected from stearic acid, myristic acid, palmitic acid, and mixtures thereof; and/or
- the further metallic salt of a fatty acid is selected from calcium stearate, zinc stearate, and mixtures thereof; and/or
- the fatty acid ester is selected from glyceride esters, preferably selected from glyceryl monostearate, glyceryl tribehenate, glyceryl dibehenate, and mixtures thereof; and sugar esters, preferably selected from sorbitan monostearate, sucrose monopalmitate, and mixtures thereof; and mixtures thereof; and/or
- the inorganic material is talc; and/or
- the polymeric lubricant is macrogol.

According to the invention, the lubricant component comprises or essentially consists of magnesium stearate.

In a particularly preferred embodiment, the polymer component comprises or essentially consists of ethylcellulose and the lubricant component comprises or essentially consists of magnesium stearate.

The weight content of the lubricant component in the coated particles and in the controlled release coating material, respectively, is comparatively high. In conventional dosage forms, lubricants are usually employed in substantially lower amounts.

It has been surprisingly found that the lubricant component, especially magnesium stearate, has a retardant effect when it is employed in such comparatively high concentrations in a controlled release coating material which additionally comprises a polymer component, especially ethylcellulose.

Preferably, the weight content of the lubricant component, relative to the total weight of the controlled release coating material, is at least 15 wt.-%, more preferably at least 16 wt.-%, still more preferably at least 17 wt.-%, yet more preferably at least 18 wt.-%, even more preferably at least 19 wt.-%, most preferably at least 20 wt.-%, and in particular at least 21 wt.-%.

Preferably, the weight content of the lubricant component, relative to the total weight of the controlled release coating material, is not more than 35 wt.-%, more preferably not more than 34 wt.-%, still more preferably not more than 33 wt.-%, yet more preferably not more than 32 wt.-%, even more preferably not more than 31 wt.-%, most preferably not more than 30 wt-%, and in particular not more than 29 wt.-%.

Preferably, the weight content of the lubricant component, relative to the total weight of the controlled release coating material, is within the range of 25±9.0 wt.-% , more preferably 25±8.0 wt.-%, still more preferably 25±7.0 wt.-%, yet more preferably 25±6.0 wt.-%, even more preferably 25±5.0 wt.-%, most preferably 25±4.0 wt.-%, and in particular 25±3.0 wt.-%.

Preferably, the weight content of the lubricant component, relative to the total weight of the coated particles, is at least 0.1 wt.-%, more preferably at least 0.5 wt.-%, still more preferably at least 1.0 wt.-%, yet more preferably at least 1.5 wt.-%, even more preferably at least 2.0 wt.-%, most preferably at least 2.2 wt.-%, and in particular at least 2.4 wt.-%.

Preferably, the weight content of the lubricant component, relative to the total weight of the coated particles, is at least 2.6 wt.-%, or at least 2.8 wt.-%, or at least 3.0 wt.-%, more preferably at least at least 3.2 wt.-%, or at least 3.4 wt.-%, or at least 3.6 wt.-%, still more preferably at least 3.8 wt.-%, or at least 4.0 wt.-%, or at least 4.2 wt.-%, yet more preferably at least 4.4 wt.-%, or at least 4.6 wt.-%, or at least 4.8 wt.-%, even more preferably at least 5.0 wt.-%, or at least 5.2 wt.-%, most preferably at least 5.4 wt.-%, or at least 5.6 wt.-%, and in particular at least at least 5.8 wt.-%

Preferably, the weight content of the lubricant component, relative to the total weight of the coated particles, is not more than 10.0 wt.-%, or not more than 9.0 wt.-%, or not more than 8.0 wt.-%, or not more than 7.0 wt.-%, or not more than 6.0 wt.-%, more preferably not more than 5.5 wt.-%, still more preferably not more than 5.0 wt.-%, yet more preferably not more than 4.5 wt.-%, even more preferably not more than 4.0 wt.-%, most preferably not more than 3.8 wt.-%, and in particular not more than 3.6 wt.-%.

Preferably, the weight content of the lubricant component, relative to the total weight of the coated particles, is within the range of 3.0±2.5 wt.-%, more preferably 3.0±2.2 wt.-%, still more preferably 3.0±1.9 wt.-%, yet more preferably 3.0±1.6 wt.-%, even more preferably 3.0±1.3 wt.-%, most preferably 3.0±1.0 wt.-%, and in particular 3.0±0.7 wt.-%.

The lubricant component, which comprises or essentially consists of magnesium stearate, according to the invention is preferably present in form of particles that have a distinct average particle size and particle size distribution. It has been surprisingly found that the *in vitro* release profile of the dosage form according to the invention can be altered by varying the particle size of the lubricant component.

In a preferred embodiment, the lubricant component is present in form of particles having a volume mean diameter Dᵥ[4,3] within the range of 5.0±4.5 µm, or 5.0±4.0 µm, or 5.0±3.5 µm, or 5.0±3.0 µm, or 5.0±2.5 µm.

In another preferred embodiment, the lubricant component is present in form of particles having a volume mean diameter Dᵥ[4,3] within the range of 7.5±7.0 µm, or 7.5±6.5 µm, or 7.5±6.0 µm, or 7.5±5.5 µm, or 7.5±5.0 µm, or 7.5±4.5 µm, or 7.5±4.0 µm, or 7.5±3.5 µm, or 7.5±3.0 µm, or 7.5±2.5 µm.

In still another preferred embodiment, the lubricant component is present in form of particles having a volume mean diameter Dᵥ[4,3] within the range of 10±9.5 µm, or 10±9.0 µm, or 10±8.5 µm, or 10±8.0 µm, or 10±7.5 µm, or 10±7.0 µm, or 10±6.5 µm, or 10±6.0 µm, or 10±5.5 µm, or 10±5.0 µm, or 10±4.5 µm, or 10±4.0 µm, or 10±3.5 µm, or 10±3.0 µm, or 10±2.5 µm.

In yet another preferred embodiment, the lubricant component is present in form of particles having a volume mean diameter Dᵥ[4,3] within the range of 15±14.5 µm, or 15±14 µm, or 15±13.5 µm, or 15±13 µm, or 15±12.5 µm, or 15±12 µm, or 15±11.5 µm, or 15±11 µm, or 15±10.5 µm, or 15±10 µm, or 15±9.5 µm, or 15±9.0 µm, or 15±8.5 µm, or 15±8.0 µm, or 15±7.5 µm, or 15±7.0 µm, or 15±6.5 µm, or 15±6.0 µm, or 15±5.5 µm, or 15±5.0 µm, or 15±4.5 µm, or 15±4.0 µm, or 15±3.5 µm, or 15±3.0 µm, or 15±2.5 µm.

In another preferred embodiment, the lubricant component is present in form of particles having a volume mean diameter Dᵥ[4,3] within the range of 25±22.5 µm, or 25±20 µm, or 25±17.5 µm, or 25±15 µm, or 25±12.5 µm, or 25±10 µm, or 25±7.5 µm, or 25±5.

In still another preferred embodiment, the lubricant component is present in form of particles having a volume mean diameter Dᵥ[4,3] within the range of 50±45 µm, or 50±40 µm, or 50±35 µm, or 50±30 µm, or 50±25 µm, or 50±20 µm, or 50±15 µm, or 50±10 µm, or 50±5 µm.

In yet another preferred embodiment, the lubricant component is present in form of particles having a volume mean diameter Dᵥ[4,3] within the range of 75±70 µm, or 75±65 µm, or 75±60 µm, or 75±55 µm, or 75±50 µm, or 75±45 µm, or 75±40 µm, or 75±35 µm, or 75±30 µm, or 75±25 µm, or 75±20 µm, or 75±15 µm, or 75±10 µm, or 75±5 µm.

The volume mean diameter Dᵥ[4,3] is preferably determined by laser diffraction, preferably in the dry mode, preferably by means of a Malvern MasterSizer^{®}.

Principally, in addition to one or more cellulose ethers, any film-forming polymer known in the art that can modulate the release of an active agent from a pharmaceutical composition can be used in the oral pharmaceutical dosage form according to the invention. The characteristics of the polymer used in the controlled release coating material are governed by the structure, size and properties of its macromolecules. The controlled release coating material can decrease dissolution rates upon exposure to biological fluids, thereby allowing modulation of Cₘₐₓ, Tₘₐₓ and AUC or exposure.

Suitable film-forming polymers include cellulose ethers such as ethylcellulose, hydroxypropylcellulose, hydroxypropylmethylcellulose or a mixture of one or more cellulose ethers with cellulose acetate, polyvinyl acetate, povidone, cross-linked starch, cross-linked chitosan, cross-linked gelatin, cross-linked hyaluronic acid, cross-linked polyvinyl alcohol, cross-linked sodium carboxymethylcellulose, cross-linked polyvinyl pyrrolidone, carboxypolymethylene or zein.

Suitable cellulose derivatives include but are not limited to alkyl celluloses, cellulose esters, cellulose diesters, cellulose triesters, cellulose ethers, cellulose ester-ethers, cellulose acylates, cellulose diacylates, cellulose triacylates, cellulose acetates, cellulose diacetates, cellulose triacetates, cellulose acetate propionates, cellulose acetate butyrates and mixtures thereof.

An exemplary film-forming polymer is ethylcellulose, used by itself or in combination with other film-forming polymers. Ethylcellulose is generally insoluble in water and gastrointestinal fluid. It is commonly used in combination with hydroxypropyl methylcellulose and other film-formers to toughen or influence the dissolution rate of the film. Due to the solubility characteristics of ethylcellulose, this polymer can be applied as an aqueous dispersion or an organic solvent dispersion. Aqueous ethylcellulose dispersions can include dispersion agents, surfactants, such as sodium lauryl sulfate, anti-foam agents, plasticizers and solubility aids.

Dispersions of ethylcellulose are commercially available. For example, a commercially available aqueous dispersion of ethylcellulose is marketed under the trademark Aquacoat ECD^{®} (FMC Corp.). Aquacoat ECD^{®} is a water-based system that includes from 24.5-29.5% by weight ethylcellulose, 0.9%-1.7% sodium lauryl sulfate and 1.7-3.3% cetyl alcohol. Another commercially available aqueous dispersion of ethylcellulose is marketed under the trademark Surelease^{®} (Colorcon, Inc.). This product is prepared by incorporating a plasticizer, such as ammonium oleate, into the dispersion of ethylcellulose during the manufacturing process.

Organic solvent dispersions of ethylcellulose can include any suitable organic solvent. Exemplary organic solvents include alcohols, such as ethanol, propanal, and butanol, acetone, ethyl acetate and ethyl lactate. Organic solvent dispersions of ethylcellulose also can include dispersing aids, surfactants and plasticizers. The organic solvent coating systems can include water, and thus are not strictly non-aqueous. These systems are predominantly organic in nature due to the greater proportion of organic solvent in the mixed dispersions.

Another exemplary film-forming polymer is cellulose acetate, e.g. cellulose triacetate, used in combination with one or more cellulose ethers. Cellulose acetate is partially acetylated cellulose in which the acetyl content ranges from 29.0% to 44.8% corresponding to mono-, di-, and triacetate. Cellulose acetate may be employed as such or as cellulose acetate butyrate, cellulose acetate propionate, cellulose acetate phthalate, or cellulose acetate trimellitate.

Another exemplary film-forming polymer is polyvinyl acetate, used in combination with one or more cellulose ethers. Polyvinyl acetate can be formulated into aqueous dispersions, and provides excellent film-forming properties and pH-independent adjustment of release rate.

Aqueous polyvinyl acetate dispersions can include dispersion agents, surfactants, such as sodium lauryl sulfate, anti-foam agents, plasticizers and solubility aids.

Commercially available film-coating formulations containing polyvinyl acetate can be used. Exemplary commercially available formulations that can be included in the release control component include an aqueous dispersion of polyvinyl acetate stabilized with povidone and sodium lauryl sulfate, such as the commercially available formulation marketed under the trademark Kollicoat^{®} (BASF).

Exemplary plasticizers include long chain fatty alcohols such as, for example, cetyl alcohol, myristyl alcohol, and stearyl alcohol, fatty acids, such as oleic, steric, linoleic, myristic, palmitic, and lauric, fatty acid esters, such as sorbityl derivatives and glycerides, acetylated monoglycerides, acetyl glycols, acetyl tributyl citrate, glycerides such as glyceryl esters of fatty acids or hydrogenated aliphatic acids such as, for example, glyceryl monostearate, glyceryl distearate, castor oil and derivatives thereof, glyceryl esters of hydrogenated castor oil, dibutyl sebacate, dibutyl tartrate, diethyl phthalate, dimethyl phthalate, and other phthalate esters, butyl phthalyl butyl glycolate, ethyl phthalyl ethyl glycolate, glycerin, propylene glycol, tributyl citrate, triethyl citrate, triacetin, triacetin citrate, and tripropionin.

In some embodiments, besides the film-forming polymer and the optionally present excipients (dispersion agents, surfactants, anti-foam agents, plasticizers, solubility aids, and the like), the controlled release coating material preferably includes a hydrophilic pore former.

The pore former can be a water soluble material that enhances release by creating channels or passageways in the controlled release coating, or by otherwise weakening the integrity of the coating upon exposure to an environmental fluid. The pore former can be a polysaccharide such as lactose, sucrose, dextrose, mannitol, d-mannitol, alpha-d-lactose monohydrate, glucose or mixture thereof, a cellulosic material such as microcrystalline cellulose, hydroxypropylmethylcellulose or a mixture thereof, or a material such as polyvinyl alcohol. The pore former can also be a water soluble polymer such as polyethylene glycol, povidone, poloxamer and combinations thereof. The pore former can also be an organic solvent such as propylene glycol. Other materials that can be pore formers include osmagents or osmotic agents such as organic and inorganic compounds. Such agents can include salts, acids, bases, chelating agents, sodium chloride, calcium sulfate, calcium phosphate, lithium chloride, magnesium chloride, magnesium sulfate, lithium sulfate, potassium chloride, sodium sulfite, calcium bicarbonate, sodium sulfate, calcium lactate, urea, tartaric acid, raffinose, and combinations thereof.

The pore former included in the controlled release coat can be any art recognized pore forming chemical species that is compatible for use with the film-forming polymer. For example, when the film-forming polymer includes ethylcellulose, the hydrophilic pore former is selected to be compatible with ethylcellulose dispersions.

Pore formers can be water-soluble hydrophilic polymers, proteins, sugars or sugar alcohols. Exemplary pore formers include cellulose ethers, including hydroxypropyl methylcellulose, sodium alginate, polyvinylpyrrolidone, cross-linked polyvinylpyrrolidone, polyethylene oxide, polyethylene glycol, pullulan, dextran, sucrose, glucose, fructose, mannitol, lactose, mannose, galactose, and sorbitol. In a preferred embodiment, the pore former is hydroxypropyl methylcellulose.

Pore formers can be salts which are water-soluble and pharmaceutically acceptable. The cations of these salts can be alkali metals such as sodium and potassium, alkaline earth metals such as magnesium, calcium and barium, or other cations such as ammonium, ferric, etc. The anions may include 1-hydroxy-2-naphthoic acid, 2,2-dichloroacetic acid, 2-hydroxyethanesulfonic acid, 2-oxoglutaric acid, 4-acetamidobenzoic acid, 4-aminosalicylic acid, acetic acid, adipic acid, ascorbic acid (L), aspartic acid (L), benzenesulfonic acid, benzoic acid, camphoric acid (+), camphor-10-sulfonic acid (+), capric acid (decanoic acid), caproic acid (hexanoic acid), caprylic acid (octanoic acid), carbonic acid, cinnamic acid, citric acid, cyclamic acid, dodecylsulfuric acid, ethane-1,2-disulfonic acid, ethanesulfonic acid, formic acid, fumaric acid, galactaric acid, gentisic acid, glucoheptonic acid (D), gluconic acid (D), glucuronic acid (D), glutamic acid, glutaric acid, glycerophosphoric acid, glycolic acid, hippuric acid, hydrobromic acid, hydrochloric acid, isobutyric acid, lactic acid (DL), lactobionic acid, lauric acid, maleic acid, malic acid (-L), malonic acid, mandelic acid (DL), methanesulfonic acid, naphthalene-1,5-disulfonic acid, naphthalene-2-sulfonic acid, nicotinic acid, nitric acid, oleic acid, oxalic acid, palmitic acid, pamoic acid, phosphoric acid, proprionic acid, pyroglutamic acid (-L), salicylic acid, sebacic acid, stearic acid, succinic acid, sulfuric acid, tartaric acid (+L), thiocyanic acid, toluenesulfonic acid (p) and undecylenic acid.

The amount of pore former included in the controlled release coating material depends to some degree on the chemistry of the pore former selected. The relative amounts of pore former and firm-forming polymer, such as ethylcellulose, in the controlled release coating material can be varied to adjust the rate of release, with larger proportions of pore former resulting in faster release rates compared to smaller proportions of pore former. In some embodiments, the controlled release coating material contains from 60 wt.-% to 95 wt.-% of firm-forming polymer such as ethylcellulose and from 40 wt.-% to 5 wt.-% of pore former such as hydroxypropyl methylcellulose.

Preferably, the pore former is used in a range from 0.1 wt.-% to 50 wt.-%, relative to the total weight of the controlled release coating material.

In preferred embodiments, the weight content of the pore former is within the range of 1.0±0.9 wt.-%, or 1.0±0.5 wt.-%; or 2.5±2.4 wt.-%, 2.5±2.0 wt.-%, or 2.5±1.5 wt. %, or 2.5±1.0 wt. %, or 2.5±0.5 wt.-%; or 5.0±4.9 wt.-%, or 5.0±4.5 wt.-%, or 5.0±4.0 wt. %, or 5.0±3.5 wt. %, or 5.0±3.0 wt.-%, or 5.0±2.5 wt. %, or 5.0±2.0 wt.-%; or 7.5±4.9 wt.-%, or 7.5±4.5 wt.-%, or 7.5±4.0 wt.-%, or 7.5±3.5 wt.-%, or 7.5±3.0 wt.-%, or 7.5±2.5 wt.-%, or 7.5±2.0 wt.-%; or 10.0±4.9 wt.-%, or 10.0±4.5 wt.-%, or 10.0±4.0 wt.-%, or 10.0±3.5 wt.-%, or 10.0±3.0 wt.-%, or 10.0±2.5 wt.-%, or 10.0±2.0 wt.-%; or 12.5±4.9 wt.-%, or 12.5±4.5 wt.-%, or 12.5±4.0 wt.-%, or 12.5±3.5 wt.-%, or 12.5±3.0 wt.-%, or 12.5±2.5 wt.-%, or 12.5±2.0 wt.-%; or 15.0±4.9 wt.-%, or 15.0±4.5 wt.-%, or 15.0±4.0 wt.-%, or 15.0±3.5 wt.-%, or 15.0±3.0 wt.-%, or 15.0±2.5 wt.-%, or 15.0±2.0 wt.-%; or 17.5±4.9 wt.-%, or 17.5±4.5 wt.-%, or 17.5±4.0 wt.-%, or 17.5±3.5 wt.-%, or 17.5±3.0 wt.-%, or 17.5±2.5 wt.-%, or 17.5±2.0 wt.-%; or 20.0±4.9 wt.-%, or 20.0±4.5 wt.-%, or 20.0±4.0 wt.-%, or 20.0±3.5 wt.-%, or 20.0±3.0 wt.-%, or 20.0±2.5 wt.-%, or 20.0±2.0 wt.-%; or 22.5±4.9 wt.-%, or 22.5±4.5 wt.-%, or 22.5±4.0 wt.-%, or 22.5±3.5 wt.-%, or 22.5±3.0 wt.-%, or 22.5±2.5 wt.-%, or 22.5±2.0 wt.-%; or 25.0±4.9 wt.-%, or 25.0±4.5 wt.-%, or 25.0±4.0 wt.-%, or 25.0±3.5 wt.-%, or 25.0±3.0 wt.-%, or 25.0±2.5 wt.-%, or 25.0±2.0 wt.-%; or 27.5±4.9 wt.-%, or 27.5±4.5 wt.-%, or 27.5±4.0 wt.-%, or 27.5±3.5 wt.-%, or 27.5±3.0 wt.-%, or 27.5±2.5 wt.-%, or 27.5±2.0 wt.-%; or 30.0±4.9 wt.-%, or 30.0±4.5 wt.-%, or 30.0±4.0 wt.-%, or 30.0±3.5 wt.-%, or 30.0±3.0 wt.-%, or 30.0±2.5 wt.-%, or 30.0±2.0 wt.-%; or 32.5±4.9 wt.-%, or 32.5±4.5 wt.-%, or 32.5±4.0 wt.-%, or 32.5±3.5 wt.-%, or 32.5±3.0 wt.-%, or 32.5±2.5 wt.-%, or 32.5±2.0 wt.-%, in each case relative to the total weight of the controlled release coating material.

The weight ratio of the film-forming polymer such as ethylcellulose to the pore former in the controlled release coating material preferably ranges from 1: 1 to 20:1. In some embodiments, the ratio is within the range of (2.5±1.5):1, or (5.0±4.0):1, or (5.0±2.5):1, or (7.5±4.0):1, or (7.5±2.5):1, or (10.0±4.0):1, or (10.0±2.5):1, or (12.5±4.0):1, or (12.5±2.5):1, or (15.0±4.0):1, or (15.0±2.5):1.

The release profile of the Tapentadol component can be modulated by the thickness of the control release coat as well as the characteristics of the controlled release coating material.

As a further alternative to the above controlled release coating materials according to embodiments (i) to (v), the controlled release coating material may be based on a mixture of a waxy material and a hydrophilic cellullose ether. Any reference to this further alternative essentially serves information purposes.

Preferably, the waxy material is selected from hydrophobic waxy materials to provide a controlled release of the active agent over the entire small and large intestines. Waxy materials are preferably selected from materials such as carnauba wax, white wax, candelilla wax, beeswax, cetylester wax, montan wax, microcrystalline wax, lecithin, hydrogenated tallow, paraffin wax, shellac wax, paraffin soft, glyceril behenate, glycerol palmitoesterarate, glycerol diestearate, tribehenin, glycerol esters such as glyceril behenate and glyceryl palmitostearate, fatty alcohols (particularly those having 12-24 carbon atoms, such as cetyl alcohol, cetostearyl alcohol, lauryl alcohol, myristyl alcohol, stearyl alcohol, palmityl alcohol, etc.), fatty acids (particularly those having 12-24 carbon atoms, such as lauric acid, myristic acid, stearic acid, palmitic acid, etc), castor wax (i.e. hydrogenated castor-oil), C₁₆-₃₀ fatty acid triglycerides stearyl polyoxil-32 glyceride, behenoyl polyoxil-8 glyceride, lauroyl polyoxil glycerides, steral polyoxil glycerides, and mixtures thereof.

Preferably, the hydrophilic cellulose ether is selected from sodium carboxy methylcellulose, hydroxypropylcellulose, hydroxypropylmethylcellulose, hydroxyethylmethylcellulose, sodium carboxymethylcellulose, calcium carboxymethylcellulose or the mixtures thereof. Preferably, the hydrophilic cellulose ether is hydroxypropylmethylcellulose.

In a preferred embodiment, the coated particles comprise or essentially consist of a core which comprises a drug coat and a controlled release coat, wherein the drug coat comprises the Tapentadol component and wherein the controlled release coat comprises the controlled release coating material.

Preferably, when the core comprises a drug coat, the drug coat and the controlled release coat are in intimate contact with one another.

It has been surprisingly found that a subcoat between the drug coat and the controlled release coat can be omitted. Subcoats are usually necessary in order to avoid migration of the active ingredient from the inside to the outside thereby altering the release characteristics and sometimes also the stability upon storage.

Preferably, at least 80 wt.-% of the total content of the Tapentadol component, which is contained in the coated particles, is contained in the drug coat, more preferably at least 85 wt.-%, still more preferably at least 90 wt.-%, yet more preferably at least 92.5 wt.-%, even more preferably at least 80 wt.-%, most preferably at least 95 wt.-%, and in particular at least 97.5 wt.-%.

Preferably, the core comprises or essentially consists of an inert carrier material not containing a pharmacologically active ingredient. Preferably, the core comprises or essentially consists of microcrystalline cellulose.

Preferably, the weight content of the core, relative to the total weight of the coated particles, is within the range of 72±22 wt.-%, more preferably 72±19 wt.-%, still more preferably 72±16 wt.-%, yet more preferably 72±13 wt.-%, even more preferably 72±10 wt.-%, most preferably 72±7.0 wt.-%, and in particular 72±4.0 wt.-%.

In preferred embodiments, the weight content of the drug coat, relative to the total weight of the coated particles, is within the range of 10.0±8.0 wt. %, more preferably 10.0±7.0 wt.-%, still more preferably 10.0±6.0 wt.-%, yet more preferably 10.0±5.0 wt.-%, even more preferably 10.0±4.0 wt.-%, most preferably 10.0±3.0 wt.-%, and in particular 10.0±2.0 wt.-%.

In preferred embodiments, the weight content of the drug coat, relative to the total weight of the coated particles, is within the range of 12.0±8.0 wt. %, more preferably 12.0±7.0 wt.-%, still more preferably 12.0±6.0 wt.-%, yet more preferably 12.0±5.0 wt.-%, even more preferably 12.0±4.0 wt.-%, most preferably 12.0±3.0 wt.-%, and in particular 12.0±2.0 wt.-%.

In preferred embodiments, the weight content of the drug coat, relative to the total weight of the coated particles, is within the range of 14.0±8.0 wt. %, more preferably 14.0±7.0 wt.-%, still more preferably 14.0±6.0 wt.-%, yet more preferably 14.0±5.0 wt.-%, even more preferably 14.0±4.0 wt.-%, most preferably 14.0±3.0 wt.-%, and in particular 14.0±2.0 wt.-%.

In preferred embodiments, the weight content of the drug coat, relative to the total weight of the coated particles, is within the range of 16.0±8.0 wt. %, more preferably 16.0±7.0 wt.-%, still more preferably 16.0±6.0 wt.-%, yet more preferably 16.0±5.0 wt.-%, even more preferably 16.0±4.0 wt.-%, most preferably 16.0±3.0 wt.-%, and in particular 16.0±2.0 wt.-%.

In preferred embodiments, the weight content of the drug coat, relative to the total weight of the coated particles, is within the range of 18.0±8.0 wt. %, more preferably 18.0±7.0 wt.-%, still more preferably 18.0±6.0 wt.-%, yet more preferably 18.0±5.0 wt.-%, even more preferably 18.0±4.0 wt.-%, most preferably 18.0±3.0 wt.-%, and in particular 18.0±2.0 wt.-%.

In preferred embodiments, the weight content of the drug coat, relative to the total weight of the coated particles, is within the range of 20.0±8.0 wt. %, more preferably 20.0±7.0 wt.-%, still more preferably 20.0±6.0 wt.-%, yet more preferably 20.0±5.0 wt.-%, even more preferably 20.0±4.0 wt.-%, most preferably 20.0±3.0 wt.-%, and in particular 20.0±2.0 wt.-%.

In preferred embodiments, the weight content of the drug coat, relative to the total weight of the coated particles, is within the range of 25.0±8.0 wt. %, more preferably 25.0±7.0 wt.-%, still more preferably 25.0±6.0 wt.-%, yet more preferably 25.0±5.0 wt.-%, even more preferably 25.0±4.0 wt.-%, most preferably 25.0±3.0 wt.-%, and in particular 25.0±2.0 wt. %.

Preferably, the drug coat forms a layer having an average thickness of at least 1.0 µm, more preferably at least 2.0 µm, still more preferably at least 3.0 µm, yet more preferably at least 4.0 µm, even more preferably at least 5.0 µm, most preferably at least 6.0 µm, and in particular at least 7.0 µm.

Preferably, the drug coat forms a layer having an average thickness of not more than 20 µm, more preferably not more than 19 µm, still more preferably not more than 18 µm, yet more preferably not more than 17 µm, even more preferably not more than 16 µm, most preferably not more than 15 µm, and in particular not more than 14 µm.

Preferably, the drug coat forms a layer having an average thickness within the range of 11.0±9.0 µm, more preferably 11.0±8.0 µm, still more preferably 11.0±7.0 µm, yet more preferably 11.0±6.0 µm, even more preferably 11.0±5.0 µm, most preferably 11.0±4.0 µm, and in particular 11.0±3.0 µm.

The thickness of this layer can also be easily determined, e.g. based on electron micrographs.

Alternatively, the core may be an extruded or spheronized material homogeneously comprising the Tapentadol component and being coated with the controlled release coating material. According to the embodiment, the coated particles preferably do not comprise a drug coat as the Tapentadol component is distributed over the core already.

In a preferred embodiment, the coated particles according to the invention provide resistance against ethanolic dose dumping (alcohol induced dose dumping). This is preferably achieved by providing the coated particles with a tamper resistant coat comprising a tamper resistant coating material. Said tamper resistant coat may comprise a single layer or several layers that may comprise identical or different coating materials. Conventional coating materials employed for providing controlled release such as poly(meth)acrylates or pure ethylcellulose typically do not provide resistance against ethanolic dose dumping.

Therefore, another aspect of the invention relates to an oral pharmaceutical dosage form comprising a plurality of coated particles, wherein said coated particles comprise a core which comprises a Tapentadol component, wherein the coated particles are additionaly coated with a tamper resistant coating material that provides resistance against ethanolic dose dumping, and wherein under *in vitro* conditions the pharmaceutical dosage form provides controlled release of the Tapentadol component. If the coated particles are additionally coated with a tamper resistant coating material, the oral pharmaceutical dosage form is as described above, i.e. comprises a core which is coated with a controlled release coating material, wherein the controlled release coating material comprises a lubricant component, which comprises or essentially consists of magnesium stearate, and a polymer component, wherein the polymer component comprises one or more cellulose ethers.

For the purpose of the specification, resistance against ethanolic dose dumping preferably means that when being exposed to an aqueous ethanolic release medium, the dissolution profile of the Tapentadol component is not significantly accelerated compared to a corresponding non-ethanolic medium, such that after concomitant intake with ethanol, a patient is not at risk of a supratherapeutic overdose of the Tapentadol component.

In a preferred embodiment, the controlled release coating material forms a controlled release coat and the tamper resistant coating material forms a tamper resistant coat, wherein the tamper resistant coat preferably overcoats the controlled release coat.

In a preferred embodiment, the tamper resistant coating material forms a tamper resistant coat and the controlled release coating material forms a controlled release coat, wherein the controlled release coat preferably overcoats the tamper resistant coat.

In still another preferred embodiment, the tamper resistant coating material is mixed with the controlled release coating material and both materials form a coat that serves both purposes, controlled release as well as resistance against ethanolic does dumping.

In another preferred embodiment of the invention, controlled release of the Tapentadol component is achieved by means of a controlled release coat, wherein the controlled release coating material comprises a lubricant component, which comprises or essentially consists of magnesium stearate, and a polymer component, wherein the polymer component comprises one or more cellulose ethers.

Waxy or fatty materials include but are not limited to fatty acids, selected from materials such as carnauba wax, white wax, candelilla wax, beeswax, cetylester wax, montan wax, microcrystalline wax, lecithin, hydrogenated tallow, paraffin wax, shellac wax, paraffin soft, glyceril behenate, glycerol palmitoesterarate, glycerol diestearate, tribehenin, glycerol esters such as glyceril behenate and glyceryl palmitostearate, fatty alcohols (particularly those having 12-24 carbon atoms, such as cetyl alcohol, cetostearyl alcohol, lauryl alcohol, myristyl alcohol, stearyl alcohol, palmityl alcohol, etc), fatty acids (particularly those having 12-24 carbon atoms, such as lauric acid, myristic acid, stearic acid, palmitic acid, etc), castor wax (i.e. hydrogenated castor-oil), C₁₆₋₃₀ fatty acid triglycerides stearyl polyoxil-32 glyceride, behenoyl polyoxil-8 glyceride, lauroyl polyoxil glycerides, steral polyoxil glycerides, and mixtures thereof.

Fatty acids include pentanoic acid, hexanoic acid, heptanoic acid, octanoic acid, nonanoic acid, decanoic acid, undecanoic acid, dodecanoic acid, tridecanoic acid, tetradecanoic acid, pentadecanoic acid, hexadecanoic acid, heptadecanoic acid, octadecanoic acid, nonadecanoic acid, eicosanoic acid, heneicosanoic acid, docosanoic acid, tricosanoic acid, pentacosanoic acid, hexacosanoic acid, heptacosanoic acid, octacosanoic acid, nonacosanoic acid, triacontanoic acid, henatriacontanoic acid, dotriacontanoic acid, tritriacontanoic acid, tetratriacontanoic acid, pentatriacontanoic acid, hexatriacontanoic acid, myristoleic acid, palmitoleic acid, oleic acid, elaidic acid, vaccenic acid, linoleic acid, linoelaidic acid, linolenic acid, preferably alpha-linolenic acid, arachidonic acid, eicosapentaenoic acid, erucic acid, docosa hexanoic acid, stearidonic acid, docosapentaenoic acid, eicosatetraenoic acid and docosahexaenoic acid.

A skilled person recognizes that controlled release of the Tapentadol component may principally also be achieved by embedding the Tapentadol component in a controlled release matrix material, e.g. based on a fatty material, and additionally applying a controlled release coating material (controlled release coat) as described above.

In another preferred embodiment, the particles are coated with a separate controlled release coating material, wherein the controlled release coating material comprises a lubricant component, which comprises or essentially consists of magnesium stearate, and a polymer component, wherein the polymer component comprises one or more cellulose ethers. According to this embodiment, the tamper resistant coating material in the tamper resistant coat not only provides resistance against ethanolic dose dumping but additionally provides controlled release of the Tapentadol component, and/or the core may comprise a controlled release matrix material in which the Tapentadol component is embedded thereby providing controlled release of the Tapentadol component (matrix retardation).

In a preferred embodiment, the tamper resistant coating material comprises a mixture of guar gum and ethylcellulose. Such mixtures are commercially available in aqueous dispersion (Aquacoat^{®} ARC, FMC Biopolymer). According to this embodiment, ethylcellulose and guar gum counteract each other in ethanol and ethanol-free dissolution media. In the presence of ethanol, guar gum protects the soluble ethylcellulose polymer chain, maintaining intact the controlled release film. In ethanol-free media, the insoluble ethylcellulose acts as a rate-controlling hydrophobic barrier while guar gum functions as a pore former. The blend ratio of ethylcellulose and guar gum can be adjusted to achieve different drug dissolution profiles while retaining the formulation's alcohol resistance. This flexibility derives from guar gum's dual function in the coating film as pore former and alcohol-resistant element. Higher levels of guar gum thus produce faster drug dissolution as well as greater alcohol resistance. The blend ratio can therefore be optimized within a recommended range in order to reach a target release profile. Preferred ratios are between 93:7 and 85:15 of ethylcellulose : guar gum. The coating weight gain also has an influence on drug dissolution. Higher coating weight gain results in slower drug dissolution. Preferred formulations with coating weight gain of 10 wt.-% and above offer robust formulation and processing. The desired drug dissolution profile can thus be achieved by selecting the optimal combination of blend ratio and coating weight gain.

Preferred coated particles according to the invention are additionally coated with a tamper resistant coating material providing resistance against ethanolic dose dumping as well as controlled release of the Tapentadol component, but preferably are not coated with a separate controlled release coating material, i.e. do not comprise a separate controlled release coat. Preferably, the tamper resistant coating material comprises a mixture of guar gum and ethylcellulose. Preferred blend ratios of ethylcellulose : guar gum (left column) and preferred tamper resistant coat weight gains, relative to the weight of the particles not being coated with the tamper resistant coating material (first line), are summarized as embodiments A¹ to D⁶ in the table here below:

| blend ratio / weight gain | 10 to 15 wt.-% | 15 to 20 wt.-% | 20 to 25 wt.-% | 25 to 30 wt.-% | 30 to 35 wt.-% | 35 to 40 wt.-% |
|---|---|---|---|---|---|---|
| 93:7 to 91:9 | A¹ | A² | A³ | A⁴ | A⁵ | A⁶ |
| 91:9 to 89:11 | B¹ | B² | B³ | B⁴ | B⁵ | B⁶ |
| 89:11 to 87:13 | C¹ | C² | C³ | C⁴ | C⁵ | C⁶ |
| 87:13 to 85:15 | D¹ | D² | D³ | D⁴ | D⁵ | D⁶ |

In another preferred embodiment, the tamper resistant coat comprises two layers, namely an inner layer and an outer layer, whereas the inner layer comprises an alginate salt, preferably sodium alginate, and the outer layer comprises an anionic polymer, preferably an anionic acrylate polymer.

Kits for providing such layers are commercially available (Eudradec^{®} ADD, Evonik).

In a preferred embodiment, the outer layer of the tamper resistant coat comprising the anionic polymer, preferably anionic acrylate polymer is based on an anionic copolymer which is preferably based on methacrylic acid and ethyl acrylate. The ratio of the free carboxyl groups to the ester groups is preferably about 1:1 (e.g. Eudragit^{®} L30 D-55). In another preferred embodiment, the outer layer comprising the acrylate polymer is based on an anionic copolymer which is preferably based on ethyl acrylate and methyl methacrylate. The ratio of ethyl acrylate to methyl methacrylate is preferably about 2:1 (e.g. Eudragit^{®} NM 30 D).

According to this embodiment, the particles comprise three parts, whereby the core contains the Tapentadol component and is coated with a controlled release coating material, wherein the controlled release coating material comprises a lubricant component, which comprises or essentially consists of magnesium stearate, and a polymer component, wherein the polymer component comprises one or more cellulose ethers. This is surrounded by an inner layer comprising the alginate salt, preferably sodium alginate, and an outer, preferably uppermost layer comprising the anionic polymer, preferably anionic acrylate polymer. The desired drug-release profile (e.g. gastro-resistant or extended release) determines the anionic polymer, preferably anionic acrylate polymer to be implemented, its quantity, as well as the excipients it is combined with. The outer layer controls where and how the Tapentadol component is released. Upon exposure to hydro-ethanolic gastric juice, the material swells, thereby clogging the pores that are formed in the outer layer upon exposure to ethanol. In other words, ethanol triggers two opposing effects that cancel each other out: formation and clogging of pores. Consequently, these controlled-release particles have a similar delivery profile in the presence and absence of ethanol.

According to this embodiment, the coated particles comprise the core, a controlled release coating layer, wherein the controlled release coating material comprises a lubricant component, which comprises or essentially consists of magnesium stearate, and a polymer component, wherein the polymer component comprises one or more cellulose ethers; a tamper resistant coat consisting of an inner layer onto the core and an outer layer onto the inner layer. The inner and outer layers have the function of controlling the release of the Tapentadol component, which is placed in the core or at the surface of the core. The inner and outer layers have also the function of providing resistance of the release rates against the presence ethanol.

In a preferred embodiment, the coated particles essentially consist of the core, a controlled release coating layer, wherein the controlled release coating material comprises a lubricant component, which comprises or essentially consists of magnesium stearate, and a polymer component, wherein the polymer component comprises one or more cellulose ethers; a tamper resistant coat consisting of the inner layer and the outer layer and there are no further coating layers present, which would additionally control the release of the Tapentadol component.

The inner layer of the tamper resistant coat is located onto the controlled release coating layer. A sub coat may be located between the coated core and the inner layer. A sub coat may have the function to separate substances of the core from substances of the controlling layer which may be incompatible with each other. The sub coat has essentially no influence on the Tapentadol component release characteristics. Preferably there is no sub coat between the coated core and the inner layer. In this case, the inner layer of the tamper resistant coat is in direct contact with core coated with a controlled release coating material, wherein the controlled release coating material comprises a lubricant component, which comprises or essentially consists of magnesium stearate, and a polymer component, wherein the polymer component comprises one or more cellulose ethers.

The total amount of the inner layer may be in the range of 2 wt.-% to 90 wt.-%, 4 wt. % to 80 wt.-%, or 5 wt.-% to 60 wt.-% in relation to the weight of the core.

The absolute amount of polymer the inner layer may be in the case of pellets or granules with a size in the range of 50 to 1000 µm (average diameter) in the range of 2 mg/cm² to 50 mg/cm², preferably 5 mg/cm² to 40 mg/cm².

Preferably, the inner layer of the tamper resistant coat comprises at least 30 wt.-%, at least 40 wt.-%, at least 50 wt.-%, at least 60 wt. -%, at least 70 wt. -%, at least 80 wt. -%, or at least 90 wt. -% of one or more salts of alginic acid.

The salts of alginic acid may be selected from sodium alginate, potassium alginate, magnesium alginate, lithium alginate or ammonium alginate or mixtures thereof.

The salts of alginic acid used for the inner layer may preferably have a viscosity of 30 to 720 cP of a 1% aqueous solution (weight/weight).

The inner layer may comprise up to 70 wt.-%, up to 60 wt.-%, up to 50 wt.-%, or up to 40 wt.-% of physiologically acceptable excipients. The physiologically acceptable excipients in the inner layer are different from the salts of alginic acid. Preferably the inner layer comprises less than 10 wt.-%, less than 5 wt.-%, less than 1 wt.-%, or no (0%) polymers or copolymers comprising anionic side groups.

A typical inner layer may for example comprise or contain 40 to 60 wt.-% of one or more salts of alginic acid and 40 to 60 wt. -% of a glidant, for instance talc.

The outer layer is located onto the inner layer of the tamper resistant coat. A sub coat may be located between the inner layer and outer layer. The sub coat has essentially no influence on the Tapentadol component release characteristics. Preferably there is no sub coat between the core, coated with a controlled release coating material, wherein the controlled release coating material comprises a lubricant component, which comprises or essentially consists of magnesium stearate, and a polymer component, wherein the polymer component comprises one or more cellulose ethers; and the inner layer of the tamper resistant coat. In this case the outer layer is in direct contact with the inner layer.

A top coat may be located on top of the outer layer. The top coat may be preferably water-soluble, essentially water-soluble or dispersible. A top coat may have the function of coloring the coated particles or protecting from environmental influences for instance from moisture during storage. The top coat may consist out of a binder, for instance a water soluble polymer like a polysaccharide or HPMC, or a sugar compound like sucrose. The top coat may further contain physiologically acceptable excipients like pigments or glidants in high amounts. The topcoat has essentially no influence on the release characteristics. Preferably there is no top coat onto the outer layer.

The pharmaceutical dosage form may be characterized in that the core is coated with a controlled release coating material, wherein the controlled release coating material comprises a lubricant component, which comprises or essentially consists of magnesium stearate, and a polymer component, wherein the polymer component comprises one or more cellulose ethers; and additionally with a tamper resistant coat consisting of a inner layer and an outer layer.

The outer layer is comprising at least 30 wt.-%, at least 40 wt.-%, at least 50 wt.-%, at least 60 wt.-%, at least 70 wt.-%, at least 80 wt.-%, at least 90 wt.-% of one or more polymers or copolymers comprising anionic side groups. Preferably the anionic side groups are carboxylic side groups.

The outer layer may comprise up to 60 wt.-%, up to 50 wt.-%, up to 40 wt.-% of physiologically acceptable excipients. The physiologically acceptable excipients in the outer layer are different from the polymers or copolymers comprising anionic side groups. Preferably the outer layer comprises less than 10 wt.-%, less than 5 wt.-%, less than 1 wt.-% or no (0%) salts of alginic acid.

A typical outer layer may for example comprise 30 to 50 wt.-% of one or more polymers or copolymers comprising anionic side groups, for instance Eudragit^{®} L100-55, 5 to 25 wt.-% of a plasticizer, for instance triethyl citrate (TEC), and 40 wt.-% to 60 wt.-% of a glidant, for instance talc.

The total amount of the outer layer may be in the range of 2 wt.-% to 90 wt.-%, 4 wt.-% to 80 wt.-%, or 5 wt.-% to 60 wt.-% in relation to the weight of the core.

The absolute amount of polymer the outer layer may be in the case of pellets or granules with a size in the range of 50 to 1000 µm (average diameter) in the range of 2 mg/cm² to 50 mg/cm², preferably 5 mg/cm² to 40 mg/cm².

The amount of the inner layer may be preferably at least equal or higher than the amount of the outer layer.

When the inner and the outer layer are calculated together as 100 wt.-%, the amount of the inner layer may be at least 50 wt.-% or more, at least 60 wt.-% or more, at least 70 wt.-% or more, at least 80 wt.-% or more, at least 90 wt.-% or more in relation to both coating layers.

The salts of alginic acid contained in the inner layer may be selected from sodium alginate, potassium alginate, magnesium alginate, lithium alginate or ammonium alginate or any kind mixtures thereof.

The salts of alginic acid contained in the inner layer may have a viscosity of 30 to 720, preferably 40 to 450, preferably 40 to 400 or preferably 50 to 300 centipoise (cp) of a 1% aqueous solution (weight/weight). The methodology of determination of the viscosity of a polymer solution, for instance a solution of a salt of alginic acid, is well known to the skilled person. The viscosity is preferably determined according to European Pharmacopeia 7th edition, general chapter 2, methods of analysis, 2.2.8 and 2.2.10, page 27ff. The test is performed using a spindle viscometer. The viscosity of a 1% alginate solution may be determined by adding 3 g product to 250 ml of distilled water in a beaker while stirring at 800 rpm using overhead stirrer. Then additional 47 ml water was added with rinsing the walls of the beaker. After stirring for 2 hours and getting a complete solution, the viscosity is measured using a LV model of the Brookfield viscometer at 25° C. (77° F.) at 60 rpm with no. 2 spindle for samples with a viscosity of more than 100 cP and at 60 rpm with no. 1 spindle for samples with viscosity less than 100 cP. Since the weight of water is almost exactly 1 g/ml even at 25° C. "weight/weight" is regarded as equal or identical to "weight/volume" in the sense of the invention. Theoretically possible marginal differences are regarded as insignificant.

The anionic polymers contained in the outer layer may be selected from the group of (meth)acrylate copolymers or polyvinyl polymers or copolymers or celluloses. The anionic polymers or copolymers used for the outer layer are preferably not cross-linked. Anionic side groups are preferably carboxylic groups.

Suitable anionic polymer or copolymers contained in the outer layer may be carboxymethyl cellulose and its salts (CMC, Na-CMC, Blanose^{®}, Tylopur^{®}), carboxymethylethyl cellulose and its salts, cellulose acetate phthalate (CAP), cellulose acetate succinate (CAS), cellulose acetate trimelliate (CAT), hydroxypropyl methyl cellulose phthalate (HPMCP, HP50, HP55) or hydroxypropylmethyl cellulose acetate succinate (HPMCAS-LF, - MF, -HF).

Suitable polyvinyl polymers or copolymers contained in the outer layer may comprise structural units that are derived from unsaturated carboxylic acids other than acrylic acid or methacrylic acid as exemplified by polyvinylacetate-phthalate, a copolymer of vinylacetate and crotonic acid 9: 1 or polyvinylacetate-succinate.

Suitable anionic (meth)acrylate copolymers may comprise 25 to 95, preferably 40 to 95 wt.-%, in particular 60 to 40 wt.-% free-radical polymerized C₁- to C₁₈-alkyl esters, preferably C₁- to C₈- or C₁- to C₄-alkyl esters alkyl esters of acrylic or of methacrylic acid and 75 to 5 wt.-%, preferably 60 to 5 wt.-%, in particular 40 to 60 wt.-% (meth)acrylate monomers having an anionic side group, respectively a carboxylic side group. The proportions mentioned normally add up to 100 wt.-%. However it is also possible in addition, without this leading to an impairment or alteration of the essential properties, for small amounts in the region of 0 to 10 wt.-%, for example 1 to 5 wt.-% of further monomers capable of vinylic copolymerization, such as, for example, hydroxyethyl methacrylate or hydroxyethyl acrylate, to be present. It is preferred that no further monomers capable of vinylic copolymerization are present. C1- to C4-alkyl esters of acrylic or methacrylic acid are in particular methyl methacrylate, ethyl methacrylate, butyl methacrylate, methyl acrylate, ethyl acrylate and butyl acrylate. A (meth)acrylate monomer having an anionic group, respectively a carboxylic side group, may be, for example, acrylic acid, with preference for methacrylic acid.

A suitable anionic (meth)acrylate copolymer may be comprising, essentially comprising, containing or consisting of polymerized units of 10 to 40 wt.-% of acrylic or methacrylic acid; 10 to 80 wt.-% of a C₄- to C₁₈-alkyl ester of acrylic or methacrylic acid; and optionally 0 to 60 wt.-% of another vinylic monomers without cross-linking side chains. C₄- to C₁₈-alkyl ester of acrylic or methacrylic acid are preferably chosen from n-butyl methacrylate, 2-ethylhexyl acrylate, 2-ethylhexyl methacrylate, isodecyl methacrylate and lauryl methacrylate.

Another vinylic monomer is a vinylic monomer which is not acrylic or methacrylic acid or a C₄- to C₁₈-alkyl ester of acrylic or methacrylic acid. Another vinylic monomer may be preferably a C₁- to C₃-alkyl ester of acrylic or methacrylic acid, which are methyl acrylate, ethyl acrylate, propyl acrylate, methyl methacrylate, ethyl methacrylate or propyl methacrylate. Another vinylic monomer may be hydroxyethyl methacrylate, hydroxypropyl methacrylate, poly(ethylenglycol)methylether acrylat, poly(ethylenglycol)methylether methacrylat, poly(propylenglycol)methylether acrylat, poly(propylenglycol)methylether methacrylat or styrene.

In preferred embodiments, the anionic (meth)acrylate copolymer comprises polymerized units of
(i) 10 to 40 wt.-% of acrylic or methacrylic acid; 10 to 50 wt.-% of ethyl acrylate; 10 to 80 wt.-% of a C₄- to C₁₈-alkyl ester of acrylic or methacrylic acid; and optionally 0 to 20 wt.-% of methyl methacrylate; or
(ii) 20 to 40 wt.-% of methacrylic acid; 20 to 40 wt.-% of n-butyl methacrylate; and 30 to 50 wt.-% of ethyl acrylate; or
(iii) 20 to 40 wt.-% of methacrylic acid; 30 to 50 wt.-% of 2-ethylhexyl acrylate; 15 to 40 wt.-% of ethyl acrylate; and optionally 0 to 20 wt.-% of methyl methacrylate; or
(iv) 10 to 40 wt.-% of methacrylic acid; 20 to 70 wt.-% of 2-ethylhexyl methacrylate; and 10 to 50 wt.-% of ethyl acrylate; or
(v) 20 to 40 wt.-% of methacrylic acid; 20 to 50 wt.-% of 2-ethylhexyl methacrylate; and 20 to 50 wt.-% of ethyl acrylate; or
(vi) 10 to 35 wt.-% of methacrylic acid; 40 to 70 wt.-% of 2-ethylhexyl methacrylate; and 10 to 30 wt.-% of ethyl acrylate; or
(vii) 20 to 40 wt.-% of methacrylic acid; 20 to 40 wt.-% of isodecyl methacrylate; and 40 to 50 wt.-% of ethyl acrylate; or
(viii) 20 to 40 wt.-% of methacrylic acid; 20 to 40 wt.-% of lauryl methacrylate; and 30 to 50 wt.-% of ethyl acrylate.

Preferably the anionic (meth)acrylate copolymer contained in the outer layer may be characterized by a mean glass transition temperature from 25 to 120 °C or 40 to 80 °C (determined by DSC according to DIN EN ISO 11357).

Preferably the anionic (meth)acrylate copolymer contained in the outer layer may be characterized by a minimum film forming temperature of 50° C. or less (determined according to DIN ISO 2115). Preferably the anionic (meth)acrylate copolymer may be characterized by a mean molecular weight Mw is 80.000 g/mol or more (determined by gel permeation chromatography, GPC).

Further suitable anionic (meth)acrylate copolymers that may be contained in the outer layer are those composed of 40 to 60 wt.-% methacrylic acid and 60 to 40 wt.-% methyl methacrylate or 60 to 40 wt.-% ethyl acrylate (Eudragit^{®} L or Eudragit^{®} L100-55 types). Eudragit^{®} L is a copolymer of 50 wt.-% methyl methacrylate and 50 wt.-% methacrylic acid. The pH of the start of the specific Tapentadol component release in intestinal juice or simulated intestinal fluid can be stated to be pH 6.0. Eudragit^{®} L 100-55 is a copolymer of 50 wt.-% ethyl acrylate and 50 wt.-% methacrylic acid. Eudragit^{®} L30 D-55 is a dispersion comprising 30 wt.-% Eudragit^{®} L 100-55. The pH of the start of the specific Tapentadol component release in intestinal juice or simulated intestinal fluid can be stated to be pH 5.5. Likewise suitable are anionic (meth)acrylate copolymers composed of 20 to 40 wt.-% methacrylic acid and 80 to 60 wt.-% methyl methacrylate (Eudragit^{®} S type). The pH of the start of the specific Tapentadol component release in intestinal juice or simulated intestinal fluid can be stated to be pH 7.0.

Further suitable anionic (meth)acrylate copolymers that may be contained in the outer layer are those consisting of 10 to 30 wt.-% methyl methacrylate, 50 to 70 wt.-% methyl acrylate and 5 to 15 wt.-% methacrylic acid (Eudragit^{®} FS type). The pH at the start of the specific Tapentadol component release in intestinal juice or simulated intestinal fluid can be stated to be pH 7.0. Eudragit^{®} FS is a copolymer of 25 wt.-% methyl methacrylate, 65 wt.-% methyl acrylate and 10 wt.-% methacrylic acid. Eudragit^{®} FS 30 D is a dispersion comprising 30 wt.-% Eudragit^{®} FS.

Additionally suitable anionic polymers that may be contained in the outer layer are copolymers composed of 20 to 34 wt.-% methacrylic acid and/or acrylic acid; 20 to 69 wt.-% methyl acrylate; and 0 to 40 wt.-% ethyl acrylate and/or where appropriate 0 to 10 wt.-% further monomers without cross-linking side chains capable of vinylic copolymerization; with the proviso that the glass transition temperature of the copolymer according to ISO 11357-2, subsection 3.3.3 (midpoint temperature Tmg), is not more than 60° C. This (meth)acrylate copolymer is particularly suitable, because of its good elongation at break properties, for compressing pellets to tablets.

Additionally suitable anionic polymers that may be contained in the outer layer are copolymers composed of 20 to 33 wt.-% methacrylic acid and/or acrylic acid; 5 to 30 wt.-% methyl acrylate; and 20 to 40 wt.-% ethyl acrylate; and more than 10 to 30 wt.-% butyl methacrylate and where appropriate 0 to 10 wt.-% further monomers without cross-linking side chains capable of vinylic copolymerization, where the proportions of the monomers add up to 100 wt.-%, with the proviso that the glass transition temperature of the copolymer according to ISO 11357-2, subsection 3.3.3 (midpoint temperature Tmg), is 55 to 70° C. Copolymers of this type are particularly suitable, because of its good mechanical properties, for compressing pellets to tablets.

The abovementioned copolymers are composed in particular of free-radical polymerized units of 20 wt.-% to 33 wt.-%, preferably 25 wt.-% to 32 wt. -%, particularly preferably 28 wt.-% to 31 wt.-% methacrylic acid or acrylic acid, with preference for methacrylic acid; 5 wt.-% to 30 wt.-%, preferably 10 wt.-% to 28 wt.-%, particularly preferably 15 wt.-% to 25 wt.-% methyl acrylate; 20 wt.-% to 40 wt.-%, preferably 25 wt.-% to 35 wt.-%, particularly preferably 18 wt.-% to 22 wt.-% ethyl acrylate, and more than 10 wt.-% to 30 wt.-%, preferably 15 wt.-% to 25 wt.-%, particularly preferably 18 to 22 wt.-% butyl methacrylate; where the monomer composition is chosen so that the glass transition temperature of the copolymer is from 55 °C to 70 °C, preferably 59 °C to 66 °C, particularly preferably 60 °C to 65° C.

Glass transition temperature means in this connection in particular the midpoint temperature Tmg according to ISO 11357-2, subsection 3.3.3. Measurement takes place without added plasticizer, with residual monomer contents (REMO) of less than 100 ppm, with a heating rate of 10° C./min and under a nitrogen atmosphere. The copolymer preferably consists essentially to exclusively of 90 wt.-%, 95 wt.-%, or 99 wt.-% to 100 wt.-% of the monomers methacrylic acid, methyl acrylate, ethyl acrylate and butyl methacrylate in the ranges of amounts indicated above.

However, it is possible, without this necessarily leading to an impairment of the essential properties, for small amounts in the range from 0 to 10 wt. -%, e.g. 1 wt. -% to 5 wt. -% of further monomers capable of vinylic copolymerization additionally to be present, such as, for example, methyl methacrylate, butyl acrylate, hydroxyethyl methacrylate, vinylpyrrolidone, vinylmalonic acid, styrene, vinyl alcohol, vinyl acetate and/or derivatives thereof.

The coated particles may comprise physiologically acceptable excipients selected from the group of antioxidants, brighteners, binding agents, flavouring agents, flow aids, fragrances, glidants, penetration-promoting agents, pigments, plasticizers, polymers, different from salts of alginic acid and different from the water-insoluble polymers or cellulosic polymers, pore-forming agents or stabilizers or combinations thereof. The physiologically acceptable excipients may be comprised in the core and/or in the inner layer and/or in the outer layer. The inner and/or the outer layer may comprise up to 60 wt.-%, up to 50 wt.-%, up to 40 wt.-% of physiologically acceptable excipients.

The coated particles as disclosed herein may be further coated with a sub coat or a top coat or both.

A sub coat may be located between the core coated with a controlled release coating material, wherein the controlled release coating material comprises a lubricant component, which comprises or essentially consists of magnesium stearate, and a polymer component, wherein the polymer component comprises one or more cellulose ethers; and the inner layer. A sub coat may have the function to separate substances of the core from substances of the controlling layer which may be incompatible with each other. The sub coat has essentially no influence on the Tapentadol component release characteristics. A subcoat is preferably essentially water-soluble, for instance it may consist of substances like hydroxypropylmethylcellulose (HPMC) as a film former. The average thickness of the subcoat layer is very thin, for example not more than 15 µm, preferably not more than 10 µm.

A top coat may be located on top of the outer layer. A top coat is also preferably essentially water soluble. A top coat may have the function of colouring the pharmaceutical or nutraceutical form or protecting from environmental influences for instance from moisture during storage. The top coat may consist out of a binder, for instance a water soluble polymer like a polysaccharide or HPMC, or a sugar compound like sucrose. The top coat may further contain excipients like pigments or glidants in high amounts. The topcoat has essentially no influence on the release characteristics.

A suitable process for producing the pharmaceutical dosage form as disclosed in here may be by forming the core comprising the Tapentadol component by direct compression, compression of dry, wet or sintered granules, by extrusion and subsequent rounding off, by wet or dry granulation, by direct pelleting or by binding powders onto Tapentadol component-free beads or neutral cores or Tapentadol component-containing particles and by applying the inner coating layer and the outer coating layer in the form of aqueous dispersions or organic solutions in spray processes or by fluidized bed spray granulation.

Preferred coated particles according to the invention, i.e. coated with a controlled release coating material, wherein the controlled release coating material comprises a lubricant component, which comprises or essentially consists of magnesium stearate, and a polymer component, wherein the polymer component comprises one or more cellulose ethers, are additionally coated with a tamper resistant coating material providing resistance against ethanolic dose dumping as well as controlled release of the Tapentadol component. Preferably, the tamper resistant coat comprises an inner layer and an outer layer, whereas the inner layer comprises sodium alginate (or another alginate salt) and the outer layer comprises an anionic acrylate copolymer that is either based on methacrylic acid and ethyl acrylate, or that is based on ethyl acrylate and methyl methacrylate.

Preferably, the tamper resistant coating material forms one or more layers (tamper resistant coat) having an overall average thickness of at least 1.0 µm, more preferably at least 2.0 µm, still more preferably at least 3.0 µm, yet more preferably at least 4.0 µm, even more preferably at least 5.0 µm, most preferably at least 6.0 µm, and in particular at least 7.0 µm.

Preferably, the tamper resistant coating material forms one or more layers (tamper resistant coat) having an overall average thickness of not more than 20 µm, more preferably not more than 19 µm, still more preferably not more than 18 µm, yet more preferably not more than 17 µm, even more preferably not more than 16 µm, most preferably not more than 15 µm, and in particular not more than 14 µm.

Preferably, the tamper resistant coating material forms one or more layers (tamper resistant coat) having an overall average thickness within the range of 11.0±9.0 µm, more preferably 11.0±8.0 µm, still more preferably 11.0±7.0 µm, yet more preferably 11.0±6.0 µm, even more preferably 11.0±5.0 µm, most preferably 11.0±4.0 µm, and in particular 11.0±3.0 µm.

The overall thickness of this layer or layers can be easily determined, e.g. based on electron micrographs.

Preferably, the tamper resistant coating material i.e. the tamper resistant coat, forms the outer surface of the coated particles. Typically, the total surface of the coated particles is additionaly coated with the tamper resistant coating material such that the particles are completely covered by and encapsulated with the tamper resistant coating material, respectively.

In a preferred embodiment, the tamper resistant coating material is coated with one or more layers of one or more different coating materials.

In another preferred embodiment, the tamper resistant coating material forms the outer coating (outer surface) of the coated particles, i.e. is exposed to the environment.

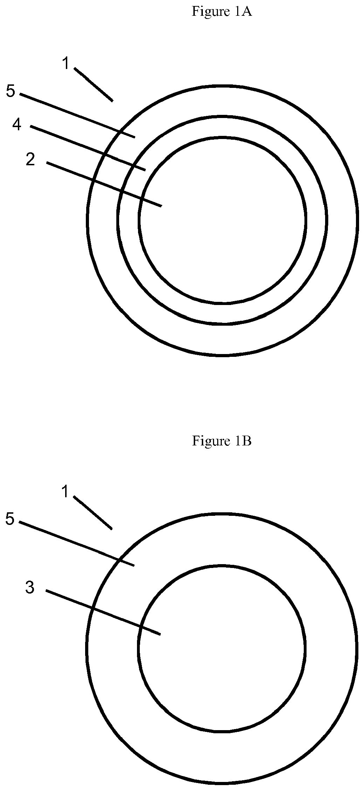

Coated particle are schematically illustrated in Figures 1A to 7B.

Figure 1A schematically illustrates a coated particle (1) comprising an inert core (2) essentially comprising no Tapentadol component (e.g. nonpareil). The inert core (2) is coated with a drug coat (4) comprising the Tapentadol component and overcoated with a controlled release coat (5) comprising the controlled release coating material. Figure 1B schematically illustrates a variant of the embodiment shown in Figure 1A, wherein the coated particle (1) comprises a core (3) comprising the Tapentadol component, but no additional drug coat. The Tapentadol component is homogeneously distributed over the core (3).

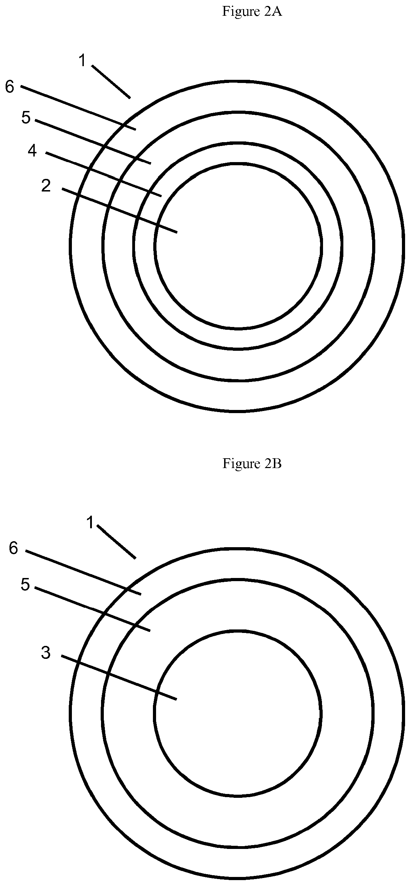

Figures 2A and 2B schematically illustrate a variant of the embodiment shown in Figures 1A and 1B, respectively, wherein in each case the controlled release coat (5) is overcoated with a tamper resistant coat (6) comprising tamper resistant coating material.

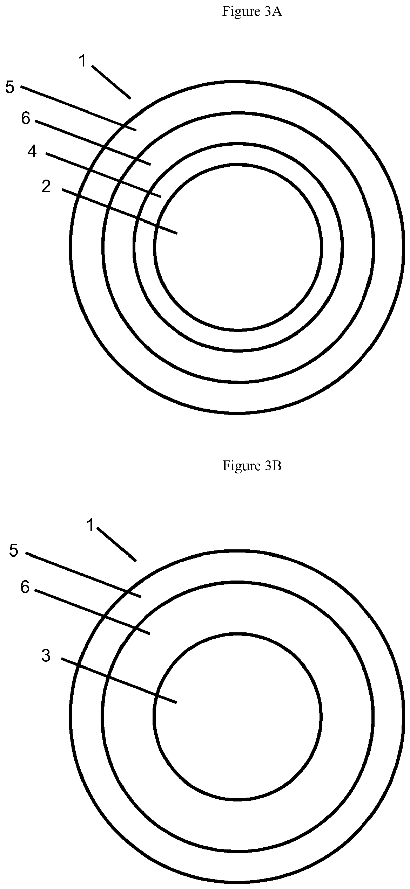

Figures 3A and 3B schematically illustrate a variant of the embodiment shown in Figures 2A and 2B, respectively, wherein in each case a tamper resistant coat (6) comprising tamper resistant coating material is overcoated with the controlled release coat (5).

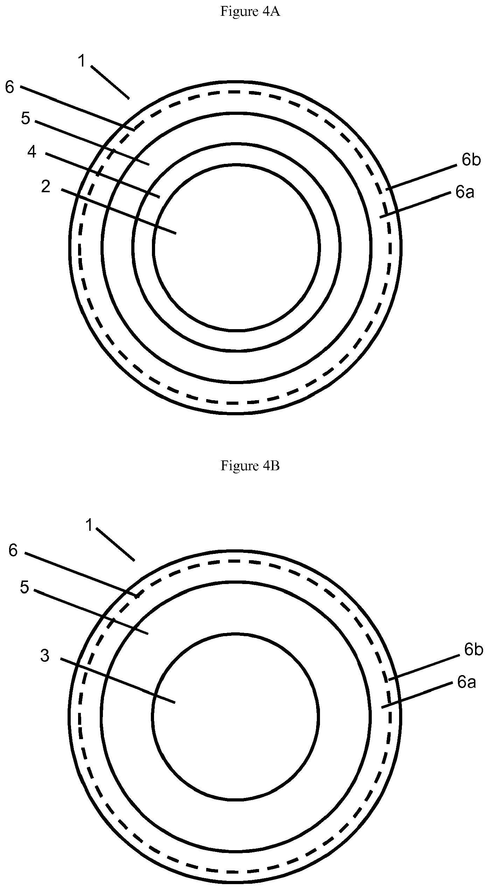

Figures 4A and 4B schematically illustrate a variant of the embodiment shown in Figures 2A and 2B, respectively, wherein the tamper resistant coat (6) is composed of an inner layer (6a) comprising alginate salt and an outer layer (6b) comprising acrylate polymer.

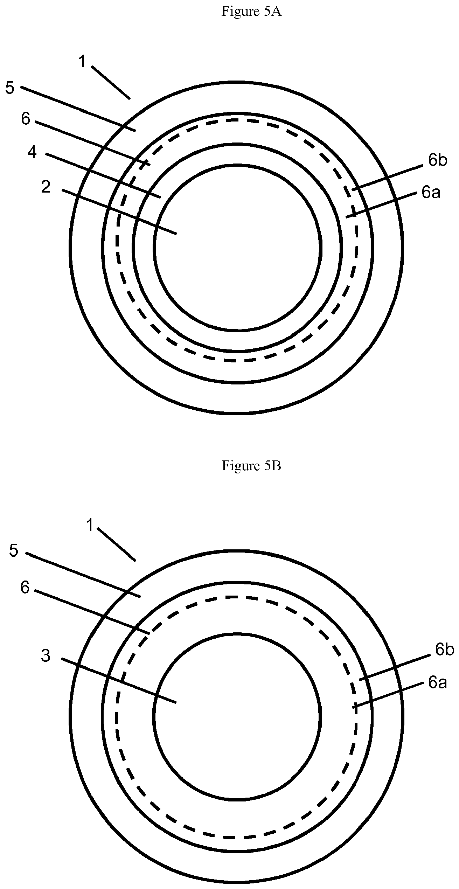

Figures 5A and 5B schematically illustrate another variant of the embodiment shown in Figures 4A and 4B, respectively, wherein in each case a tamper resistant coat (6), which is composed of an inner layer (6a) comprising alginate salt and an outer layer (6b) comprising acrylate polymer, is overcoated with the controlled release coat (5).

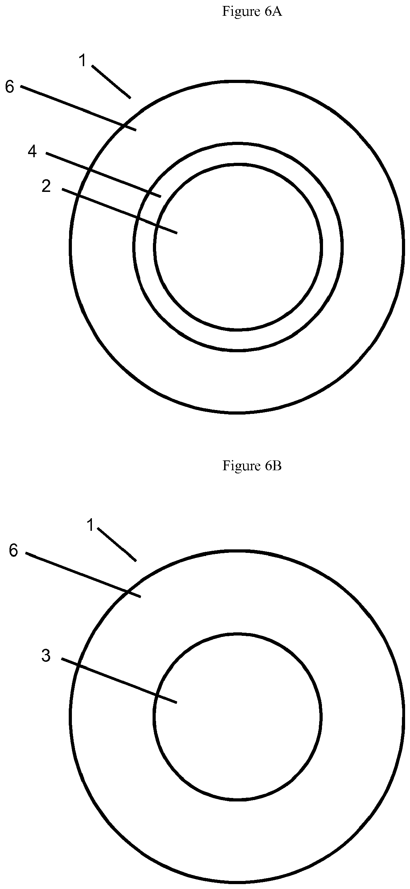

Figures 6A and 6B are not encompassed by the wording of the claims. They schematically illustrate a variant of the embodiment shown in Figures 2A and 2B, respectively, wherein the tamper resistant coat (6) provides controlled release of the Tapentadol component such that no additional controlled release coat (5) is present.

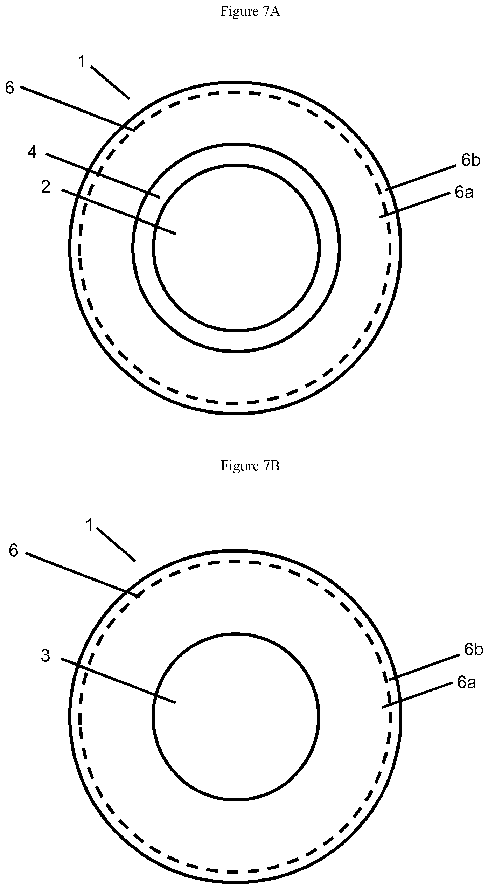

Figures 7A and 7B are not encompassed by the wording of the claims. They schematically illustrate a variant of the embodiment shown in Figures 4A and 4B, respectively, wherein the tamper resistant coat (6) provides controlled release of the Tapentadol component such that no additional controlled release coat (5) is present.

In a preferred embodiment, the coated particles comprise or essentially consist of a core which comprises a drug coat, a controlled release coat, and a tamper resistant coat, wherein the drug coat comprises the Tapentadol component, wherein the controlled release coat comprises the controlled release coating material, and wherein the tamper resistant coat comprises the tamper resistant coating material.

Preferably, the drug coat and the controlled release coat are in intimate contact with one another, and/or the controlled release coat and the tamper resistant coat are in intimate contact with one another.

Preferably, the weight content of the tamper resistant coating material, relative to the total weight of the coated particles, is not more than 45.5 wt.-% or not more than 49 wt.-%, more preferably not more than 47.5 wt.-% or not more than 45 wt.-%, still more preferably not more than 42.5 wt.-% or not more than 40 wt.-%, yet more preferably not more than 37.5 wt.-% or not more than 35 wt.-%, even more preferably not more than 32 wt. -% or not more than 31 wt.-%, most preferably not more than 30 wt. -% or not more than 29 wt. -%, and in particular not more than 28 wt.-% or not more than 27 wt.-%.

Preferably, the weight content of the tamper resistant coating material, relative to the total weight of the coated particles, is not more than 26 wt.-% or not more than 25 wt.-%, more preferably not more than 24 wt.-% or not more than 23 wt.-%, still more preferably not more than 22 wt. -% or not more than 21 wt. -%, yet more preferably not more than 20 wt.-% or not more than 19 wt.-%, even more preferably not more than 18 wt.-% or not more than 17 wt.-%, most preferably not more than 16 wt.-% or not more than 15 wt.-%, and in particular not more than 14 wt.-% or not more than 13 wt.-%.

Preferably, the weight content of the tamper resistant coating material, relative to the total weight of the coated particles, is within the range of from 5.0 wt.-% to 21 wt.-%, more preferably from 7.0 wt.-% to 19 wt.-%.

In preferred embodiments, the weight content of the tamper resistant coating material, relative to the total weight of the coated particles, is within the range of 9.0±8.0 wt.-%, or 9.0±7.0 wt.-%, or 9.0±6.0 wt.-%, or 9.0±5.0 wt.-%, or 9.0±4.0 wt.-%, more preferably 9.0±3.5 wt.-%, still more preferably 9.0±3.0 wt.-%, yet more preferably 9.0±2.5 wt.-%, even more preferably 9.0±2.0 wt.-%, most preferably 9.0±1.5 wt.-%, and in particular 9.0±1.0 wt.-% or 9.0±0.5 wt.-%.

In preferred embodiments, the weight content of the tamper resistant coating material, relative to the total weight of the coated particles, is within the range of 11.0±8.0 wt.-%, or 11.0±7.0 wt.-%, or 11.0±6.0 wt.-%, or 11.0±5.0 wt. %, or 11.0±4.0 wt. %, more preferably 11.0±3.5 wt.-%, still more preferably 11.0±3.0 wt.-%, yet more preferably 11.0±2.5 wt.-%, even more preferably 11.0±2.0 wt.-%, most preferably 11.0±1.5 wt.-%, and in particular 11.0±1.0 wt.-% or 11.0±0.5 wt. %.

In preferred embodiments, the weight content of the tamper resistant coating material, relative to the total weight of the coated particles, is within the range of 13.0±8.0 wt.-%, or 13.0±7.0 wt.-%, or 13.0±6.0 wt.-%, or 13.0±5.0 wt. %, or 13.0±4.0 wt. %, more preferably 13.0±3.5 wt. %, still more preferably 13.0±3.0 wt. %, yet more preferably 13.0±2.5 wt.-%, even more preferably 13.0±2.0 wt.-%, most preferably 13.0±1.5 wt.-%, and in particular 13.0±1.0 wt.-% or 13.0±0.5 wt. %.

In preferred embodiments, the weight content of the tamper resistant coating material, relative to the total weight of the coated particles, is within the range of 15.0±8.0 wt.-%, or 15.0±7.0 wt.-%, or 15.0±6.0 wt.-%, or 15.0±5.0 wt.-%, or 15.0±4.0 wt.-%, more preferably 15.0±3.5 wt.-%, still more preferably 15.0±3.0 wt.-%, yet more preferably 15.0±2.5 wt.-%, even more preferably 15.0±2.0 wt.-%, most preferably 15.0±1.5 wt.-%, and in particular 15.0±1.0 wt.-% or 15.0±0.5 wt. %.

In preferred embodiments, the weight content of the tamper resistant coating material, relative to the total weight of the coated particles, is within the range of 17.0±8.0 wt.-%, or 17.0±7.0 wt.-%, or 17.0±6.0 wt.-%, or 17.0±5.0 wt.-%, or 17.0±4.0 wt.-%, more preferably 17.0±3.5 wt.-%, still more preferably 17.0±3.0 wt.-%, yet more preferably 17.0±2.5 wt.-%, even more preferably 17.0±2.0 wt.-%, most preferably 17.0±1.5 wt.-%, and in particular 17.0±1.0 wt.-% or 17.0±0.5 wt. %.

In preferred embodiments, the weight content of the tamper resistant coating material, relative to the total weight of the coated particles, is within the range of 19.0±8.0 wt.-%, or 19.0±7.0 wt.-%, or 19.0±6.0 wt.-%, or 19.0±5.0 wt.-%, or 19.0±4.0 wt.-%, more preferably 19.0±3.5 wt.-%, still more preferably 19.0±3.0 wt.-%, yet more preferably 19.0±2.5 wt.-%, even more preferably 19.0±2.0 wt.-%, most preferably 19.0±1.5 wt.-%, and in particular 19.0±1.0 wt.-% or 19.0±0.5 wt. %.

In preferred embodiments, the weight content of the tamper resistant coating material, relative to the total weight of the coated particles, is within the range of 22.5±8.0 wt.-%, or 22.5±7.0 wt.-%, or 22.5±6.0 wt.-%, or 22.5±5.0 wt.-%, or 22.5±4.0 wt.-%, more preferably 22.5±3.5 wt.-%, still more preferably 22.5±3.0 wt.-%, yet more preferably 22.5±2.5 wt.-%, even more preferably 22.5±2.0 wt.-%, most preferably 22.5±1.5 wt.-%, and in particular 22.5±1.0 wt.-% or 22.5±0.5 wt.-%.

In preferred embodiments, the weight content of the tamper resistant coating material, relative to the total weight of the coated particles, is within the range of 25.0±8.0 wt.-%, or 25.0±7.0 wt.-%, or 25.0±6.0 wt.-%, or 25.0±5.0 wt.-%, or 25.0±4.0 wt.-%, more preferably 25.0±3.5 wt.-%, still more preferably 25.0±3.0 wt.-%, yet more preferably 25.0±2.5 wt.-%, even more preferably 25.0±2.0 wt.-%, most preferably 25.0±1.5 wt.-%, and in particular 25.0±1.0 wt.-% or 25.0±0.5 wt.-%.

Preferably, the coated particles have an average weight per particle of at least 5.0 µg, more preferably at least 7.5 µg, still more preferably at least 10 µg, yet more preferably at least 15 µg, even more preferably at least 20 µg, most preferably at least 25 µg, and in particular at least 30 µg.

Preferably, the coated particles have an average weight per particle of not more than 500 µg, more preferably not more than 450 µg, still more preferably not more than 400 µg, yet more preferably not more than 350 µg, even more preferably not more than 300 µg, most preferably not more than 250 µg, and in particular not more than 200 µg.

Preferably, the coated particles have an average weight per particle within the range of 100±90 µg, more preferably 100±80 µg, still more preferably 100±70 µg, yet more preferably 100±60 µg, even more preferably 100±50 µg, most preferably 100±40 µg, and in particular 100±30 µg.

The shape of the coated particles is not particularly limited. While the shape of the particles may be oval or oblong, the shape of the coated particles is preferably round and essentially spherical.

Preferably, the coated particles have an average diameter of at least 50 µm, more preferably at least 100 µm, still more preferably at least 150 µm, yet more preferably at least 200 µm, even more preferably at least 250 µm, most preferably at least 300 µm, and in particular at least 350 µm.

Preferably, the coated particles have an average diameter of not more than 1000 µm, more preferably not more than 900 µm, still more preferably not more than 800 µm, yet more preferably not more than 700 µm, even more preferably not more than 600 µm, most preferably not more than 550 µm, and in particular not more than 500 µm.

Preferably, the coated particles have an average diameter within the range of 400±300 µm, more preferably 400±260 µm, still more preferably 400±220 µm, yet more preferably 400±180 µm, even more preferably 400±140 µm, most preferably 400±100 µm, and in particular 400±60 µm.

Preferably, the plurality of coated particles has a particle size distribution such that upon sieve analysis at least 80 wt.-%, more preferably at least 82 wt.-%, still more preferably at least 84 wt. -%, yet more preferably at least 86 wt.-%, even more preferably at least 88 wt.-%, most preferably at least 90 wt.-%, and in particular at least 92 wt.-% of the coated particles have of size within the range of from 355 µm to 560 µm.

Preferably, the plurality of coated particles has a particle size distribution such that upon sieve analysis not more than 99.9 wt.-%, more preferably not more than 99.7 wt.-%, still more preferably not more than 99.5 wt.-%, yet more preferably not more than 99.3 wt.-%, even more preferably not more than 99.0 wt.-%, most preferably not more than 98.8 wt.-%, and in particular not more than 98.6 wt.-% of the coated particles have of size within the range of from 355 µm to 560 µm.

Preferably, the plurality of coated particles has a particle size distribution such that upon sieve analysis 90.0±9.9 wt.-% more preferably 90.0±9.5 wt.-%, still more preferably 90.0±9.0 wt.-%, yet more preferably 90.0±8.5 wt.-%, even more preferably 90.0±8.0 wt.-%, most preferably 90.0±7.5 wt.-%, and in particular 90.0±7.0 wt.-% of the coated particles have of size within the range of from 355 µm to 560 µm.

In a preferred embodiment, the plurality of coated particles has a particle size distribution such that upon sieve analysis
- 0.1 to 5.0 wt.-% of the coated particles have of size within the range of from 355 µm to 400 µm;
- 40 to 95 wt.-% of the coated particles have a size within the range of from 401 µm to 500 µm; and
- 5.0 to 50 wt.-% of the coated particles have a size within the range of from 501 µm to 560 µm.

Preferably, the Tapentadol component is Tapentadol free base of a physiologically acceptable salt of Tapentadol.

Preferably, the Tapentadol component is Tapentadol hydrochloride.

While it is principally possible that the dosage form according to the invention comprises one or more additional pharmacologically active ingredients, the Tapentadol component is preferably the only pharmacologically active ingredient that is contained in the dosage form.

Preferably, the weight content of the Tapentadol component, relative to the total weight of the coated particles and expressed as weight equivalents relative to Tapentadol free base, is at least 2.0 wt.-%, more preferably at least 3.0 wt.-%, still more preferably at least 4.0 wt.-%, yet more preferably at least 5.0 wt.-%, even more preferably at least 6.0 wt.-%, most preferably at least 7.0 wt.-%, and in particular at least 8.0 wt.-%.

Preferably, the weight content of the Tapentadol component, relative to the total weight of the coated particles and expressed as weight equivalents relative to Tapentadol free base, is not more than 30 wt.-%, or not more than 27.5 wt.-%, or not more than 25 wt.-%, or not more than 22.5 wt.-%, or not more than 20 wt.-% , more preferably not more than 19 wt.-%, still more preferably not more than 18 wt.-%, yet more preferably not more than 17 wt.-%, even more preferably not more than 16 wt.-%, most preferably not more than 15 wt.-%, and in particular not more than 14 wt. %.

Preferably, the weight content of the Tapentadol component, relative to the total weight of the coated particles and expressed as weight equivalents relative to Tapentadol free base, is within the range of 11.0±8.0 wt.-%, more preferably 11.0±7.0 wt.-%, still more preferably 11.0±6.0 wt.-%, yet more preferably 11.0±5.0 wt.-%, even more preferably 11.0±4.0 wt.-%, most preferably 11.0±3.0 wt.-%, and in particular 11.0±2.0 wt.-%.

The dosage form according to the invention may contain excipients that are conventionally used for the manufacture of oral dosage forms.

Pharmaceutical excipients are known to the skilled person (cf. e.g. R. C. Rowe et al., Handbook of Pharmaceutical Excipients, Pharmaceutical Press; 6th edition 2009; E.-M. Hoepfner et al., Fiedler--Encyclopedia of Excipients, Editio Cantor, 6th edition 2008).

For the purpose of the specification, a "pharmaceutical excipient" is preferably to be regarded as any pharmacologically inactive substance typically used as a carrier for the active ingredients of a medication. Typical examples of pharmaceutical excipients include but are not limited to antiadherents, binders, coating materials, disintegrants, fillers, diluents, flavours, colorants, glidants, lubricants, preservatives, sorbents, sweeteners, and the like. Binders and glidants are preferred.

In a preferred embodiment, the dosage form comprises a binder, preferably hydroxypropylmethylcellulose (hypromellose). Other suitable binders include but are not limited to saccharides and their derivatives such as disaccharides (e.g. sucrose or lactose), polysaccharides and their derivatives (e.g. starches, cellulose or modified cellulose such as microcrystalline cellulose and cellulose ethers such as hydroxypropyl cellulose (HPC)), sugar alcohols such as xylitol, sorbitol or maltitol; proteins such as gelatin; synthetic polymers such as polyvinylpyrrolidone (PVP) or polyethylene glycol (PEG). Preferably, the binder is contained in the coated particles, more preferably in a drug coat also comprising the Tapentadol component.

Preferably, the weight content of the binder, relative to the total weight of the coated particles, is at least 0.01 wt.-%, more preferably at least 0.05 wt.-%, still more preferably at least 0.1 wt.-%, yet more preferably at least 0.2 wt.-%, even more preferably at least 0.3 wt.-%, most preferably at least 0.4 wt.-%, and in particular at least 0.5 wt.-%.

Preferably, the weight content of the binder, relative to the total weight of the coated particles, is not more than 5.0 wt.-%, more preferably not more than 4.5 wt.-%, still more preferably not more than 4.0 wt.-%, yet more preferably not more than 3.5 wt.-%, even more preferably not more than 3.0 wt.-%, most preferably not more than 2.5 wt.-%, and in particular not more than 2.0 wt. %.

Preferably, the weight content of the binder, relative to the total weight of the coated particles, is within the range of 0.65±0.60 wt.-%, more preferably 0.65±0.55 wt.-%, still more preferably 0.65±0.50 wt.-%, yet more preferably 0.65±0.45 wt.-%, even more preferably 0.65±0.40 wt.-%, most preferably 0.65±0.35 wt.-%, and in particular 0.65±0.30 wt.-%.

In a preferred embodiment, the dosage form comprises a glidant, preferably silicon dioxide. Other suitable glidants include but are not limited to starch, talc, silaceous material like colloidal silica, pyrogenic silica, hydrated sodium silicoaluminate, and magnesium carbonate. Preferably, the glidant is contained in the coated particles, more preferably in a drug coat also comprising the Tapentadol component.

Preferably, the weight content of the glidant, relative to the total weight of the coated particles, is at least 0.01 wt.-%, more preferably at least 0.05 wt.-%, still more preferably at least 0.1 wt.-%, yet more preferably at least 0.2 wt.-%, even more preferably at least 0.3 wt.-%, most preferably at least 0.4 wt.-%, and in particular at least 0.5 wt.-%.

Preferably, the weight content of the glidant, relative to the total weight of the coated particles, is not more than 5.0 wt.-%, more preferably not more than 4.5 wt.-%, still more preferably not more than 4.0 wt.-%, yet more preferably not more than 3.5 wt.-%, even more preferably not more than 3.0 wt.-%, most preferably not more than 2.5 wt-%, and in particular not more than 2.0 wt.-%.

Preferably, the weight content of the glidant, relative to the total weight of the coated particles, is within the range of 0.65±0.60 wt.-%, more preferably 0.65±0.55 wt.-%, still more preferably 0.65±0.50 wt.-%, yet more preferably 0.65±0.45 wt.-%, even more preferably 0.65±0.40 wt.-%, most preferably 0.65±0.35 wt.-%, and in particular 0.65±0.30 wt.-%.

The dosage form according to the invention may principally be any type of dosage form suitable for oral administration. Examples include but are not limited to tablets, coated tablets, coated minitablets, coated pellets, coated granules, powders, suspensions, dragees, capsules or sachets filled with coated pellets or with powder or with granules, and the like.

The term coated tablet includes pellet-containing tablets or compressed tablets and is well known to a skilled person. Such a tablet may have a size of around 5 to 25 mm for instance. Usually, defined pluralities of small Tapentadol component containing pellets are compressed therein together with binding excipients to give the well known tablet form. After oral ingestion and contact with the body fluid the tablet form is disrupted and the pellets are set free. The compressed tablet combines the advantage of the single dose form for ingestion with the advantages of a multiple forms, for instance the dosage accuracy.

The term coated minitablet is well known to the skilled person. A minitablet is smaller than the traditional tablet and may have a size of around 1 to 4 mm. The minitablet is, like a pellet, a single dosage form to be used in multiple dosages. In comparison to pellets, which may be in the same size, minitablets usually have the advantage of having more regular surfaces which can be coated more accurately and more uniformly. Minitablets may be provided enclosed in capsules, such as gelatine capsules. Such capsules disrupt after oral ingestion and contact with the gastric or intestinal fluids and the minitablets are set free. Another application of minitablets is the individual fine adjustment of the Tapentadol component dosage. In this case the patient may ingest a defined number of minitablets directly which matches to the severe of the decease to cure but also to his individual body weight. A minitablet is different from pellet-containing compressed tablet as discussed above.

The term sachet is well known to the skilled person. It refers to small sealed package which contains the Tapentadol component in particle containing form. The sachet itself is only the package form is not intended to be ingested. The content of the sachet may be dissolved in water or as an advantageous feature may be soaked or ingested directly without further liquid. The latter is advantageous feature for the patient when the dosage form shall be ingested in a situation where no water is available. The sachet is an alternative dosage form to tablets, minitablets or capsules.

In a preferred embodiment, the dosage form according to the invention is provided in form of a sachet comprising the plurality of coated particles.

In another preferred embodiment, the dosage form according to the invention is provided in form of a MUPS (multiple unit pellet system) formulation, wherein the plurality of coated particles is contained in a matrix material and compressed to a tablet.

In still another preferred embodiment, the dosage form according to the invention is provided in form of a filled capsule comprising the plurality of coated particles.

Preferably, the filled capsule essentially consists of the capsule and the plurality of coated particles.

In a particularly preferred embodiment, the capsule is a sprinkle capsule. Sprinkle capsules are patient-centric capsules specifically designed to meet the needs of a rapidly growing number of patients that have difficulty swallowing. Sprinkle capsules have an innovative closure that needs less force to open so it can be opened much easier and safer. Based on the trusted design, it can be filled on the same machines as traditional capsules but will remain in place during packaging and transportation. Sprinkle capsules are commercially available (e.g. Coni-Snap^{®}).

The dosage form according to any of the invention provides prolonged release of the Tapentadol component.

In a preferred embodiment, the dosage form according to the invention provides an *in vitro* release profile measured with the paddle method according to Ph. Eur. at 50 rpm in 900 ml phosphate buffer pH 6.8 and 37 °C, such that
- after 60 min 15±10 wt.-%; more preferably 15±8.0 wt.-%; still more preferably 15±6.0 wt.-%;
- after 120 min 50±30 wt.-%; more preferably 50±25 wt.-%; still more preferably 50±15 wt.-%;
- after 240 min 70±20 wt.-%; more preferably 70±15 wt.-%; still more preferably 70±10 wt.-%;
- after 360 min 80±15 wt.-%; more preferably 80±12.5 wt.-%; still more preferably 80±10 wt.-%; and
- after 720 min 90±10 wt.-%; more preferably 90±7.5 wt.-%; still more preferably 90±5.0 wt.-%;
of the Tapentadol component that was originally contained in the dosage form have been released.

In another preferred embodiment, the dosage form according to the invention provides an *in vitro* release profile measured with the paddle method according to Ph. Eur. at 50 rpm in 900 ml phosphate buffer pH 6.8 and 37 °C, such that
- after 60 min 3.2±3.0 wt.-%; more preferably 3.2±3.0 wt.-%; still more preferably 3.2±3.0 wt.-%;
- after 120 min 35±30 wt.-%; more preferably 35±30 wt.-%; still more preferably 35±30 wt.-%;
- after 240 min 65±30 wt.-%; more preferably 65±30 wt.-%; still more preferably 65±30 wt.-%;
- after 360 min 76±14 wt.-%; more preferably 76±14 wt.-%; still more preferably 76±14 wt.-%; and
- after 720 min 89±11 wt.-%; more preferably 89±11 wt.-%; still more preferably 89±11 wt.-%;
of the Tapentadol component that was originally contained in the dosage form have been released.

The volume of the phosphate buffer of 900 ml may be reduced to 500 ml when the dose strength is reduced as well. In particular, when the dose strength is 25 mg, a buffer volume of 900 ml is preferred, whereas when the dose strength is 6.25 mg, a buffer volume of 500 ml is preferred.

In a preferred embodiment of the dosage form according to the invention, the dose of the Tapentadol component, expressed as weight equivalents relative to Tapentadol free base, is within the range of 25±5 mg.

According to this preferred embodiment, the number of the plurality of coated particles that is contained in the dosage form, e.g. in the capsule, is preferably within the range from 1000 to 1500.

According to this embodiment, the dosage form according to the invention preferably provides under fasted conditions mean (± standard deviation) pharmacokinetic parameters of
- AUC within the range of 65.1±17.6 h·ng/ml; more preferably 65.1±12.3 h-ng/ml; and/or
- Cₘₐₓ within the range of 5.22±1.83 ng/ml; more preferably 5.22±1.28 ng/ml; and/or
- tₘₐₓ within the range of 5.61±0.783 h; more preferably 5.61±0.548 h.

Alternatively, according to this embodiment, the dosage form according to the invention preferably provides under fasted conditions mean (± standard deviation) pharmacokinetic parameters of
- AUC within the range of 65.1 ± 17.6 h-ng/ml; and/or
- Cₘₐₓ within the range of 5.22 ± 1.83 ng/ml; and/or
- tₘₐₓ within the range of 5.61 ± 0.783 h.

According to this embodiment, the dosage form according to the invention preferably provides under fed status conditions mean (± standard deviation) pharmacokinetic parameters of
- AUC within the range of 101±21.1 h·ng/ml; more preferably 101±14.8 h-ng/ml; and/or
- Cₘₐₓ within the range of 10.1±2.37 ng/ml; more preferably 10.1±1.66 ng/ml; and/or
- tₘₐₓ within the range of 4.79±0.658 h; more preferably 4.79±0.460 h.

Alternatively, according to this embodiment, the dosage form according to the invention preferably provides under fed status conditions mean (± standard deviation) pharmacokinetic parameters of
- AUC within the range of 102 ± 21.0 h·ng/ml; and/or
- Cₘₐₓ within the range of 10.2 ± 2.39 ng/ml; and/or
- tₘₐₓ within the range of 4.78 ± 0.672 h.

In another preferred embodiment of the dosage form according to the invention, the dose of the Tapentadol component, expressed as weight equivalents relative to Tapentadol free base, is within the range of 50±5 mg.

According to this preferred embodiment, the number of the plurality of coated particles that is contained in the dosage form, e.g. in the capsule, is preferably within the range from 2000 to 3000.

According to this embodiment, the dosage form according to the invention preferably provides under fasted conditions mean (± standard deviation) pharmacokinetic parameters of
- AUC within the range of 130.2±35.2 h·ng/ml; more preferably 130.2±24.6 h-ng/ml; and/or
- Cₘₐₓ within the range of 10.44±3.66 ng/ml; more preferably 10.44±2.56 ng/ml.

In still another preferred embodiment of the dosage form according to the invention, the dose of the Tapentadol component, expressed as weight equivalents relative to Tapentadol free base, is within the range of 100±5 mg.

According to this preferred embodiment, the number of the plurality of coated particles that is contained in the dosage form, e.g. in the capsule, is preferably within the range from 3000 to 4500.

According to this embodiment, the dosage form according to the invention preferably provides under fasted conditions mean (± standard deviation) pharmacokinetic parameters of
- AUC within the range of 260.4±70.4 h-ng/ml; more preferably 260.4±49.3 h-ng/ml; and/or
- Cₘₐₓ within the range of 20.9±7.32 ng/ml; more preferably 20.9±5.12 ng/ml.

In another preferred embodiment of the dosage form according to the invention, the dose of the Tapentadol component, expressed as weight equivalents relative to Tapentadol free base, is within the range of 150±5 mg.

In another preferred embodiment of the dosage form according to the invention, the dose of the Tapentadol component, expressed as weight equivalents relative to Tapentadol free base, is within the range of 200±5 mg.

In another preferred embodiment of the dosage form according to the invention, the dose of the Tapentadol component, expressed as weight equivalents relative to Tapentadol free base, is within the range of 250±5 mg.

In a preferred embodiment, the dosage form according to the invention comprises the Tapentadol component as the only pharmacologically active ingredient that is contained in the pharmaceutical dosage form. In a preferred embodiment, the Tapentadol component is exclusively provided in form of the coated particles according to the invention as described above. In another preferred embodiment, the pharmaceutical dosage form contains the coated particles according to the invention as described above as well as subunits which differ from the coated particles according to the invention and which contain Tapentadol component as well. For example, said subunits may provide a different *in vitro* release profile, e.g. immediate release, such that the overall release profile of the Tapentadol component from the dosage form, i.e. from the coated particles and from the subunits, may be modified, e.g. bimodal.

In further preferred embodiments, the dosage form according to the invention provides preferably under fasted conditions a mean value for AUC within the range of 150±100 h-ng/ml; or 200±150 h-ng/ml, more preferably 200±100 h·ng/ml; or 250±200 h·ng/ml, more preferably 250±150 h·ng/ml, still more preferably 250±100 h-ng/ml; or 300±250 h·ng/ml, more preferably 300±200 h-ng/ml, still more preferably 300±150 h-ng/ml, yet more preferably 300±150 h-ng/ml; or 400±250 h-ng/ml, more preferably 400±200 h·ng/ml, still more preferably 400±150 h·ng/ml, yet more preferably 400±150 h-ng/ml; or 500±250 h·ng/ml, more preferably 500±200 h-ng/ml, still more preferably 500±150 h·ng/ml, yet more preferably 500±150 h·ng/ml; or 600±250 h-ng/ml, more preferably 600±200 h·ng/ml, still more preferably 600±150 h·ng/ml, yet more preferably 600±150 h·ng/ml; or 700±250 h·ng/ml, more preferably 700±200 h-ng/ml, still more preferably 700±150 h·ng/ml, yet more preferably 700±150 h·ng/ml; or 800±250 h·ng/ml, more preferably 800±200 h·ng/ml, still more preferably 800±150 h·ng/ml, yet more preferably 800±150 h·ng/ml; or 900±250 h·ng/ml, more preferably 900±200 h·ng/ml, still more preferably 900±150 h-ng/ml, yet more preferably 900±150 h·ng/ml.

In further preferred embodiments, the dosage form according to the invention provides preferably under fasted conditions a mean value for Cₘₐₓ within the range of 5.0±2.5 ng/ml; or 7.5±5.0 ng/ml; more preferably 7.5±2.5 ng/ml; or 10.0±7.5 ng/ml, more preferably 10.0±5.0 ng/ml, still more preferably 10.0±2.5 ng/ml; or 12.5±10.0 ng/ml, more preferably 12.5±7.5 ng/ml, still more preferably 12.5±5.0 ng/ml, yet more preferably 12.5±2.5 ng/ml; or 15.0±10.0 ng/ml, more preferably 15.0±7.5 ng/ml, still more preferably 15.0±5.0 ng/ml, yet more preferably 15.0±2.5 ng/ml; or 17.5±10.0 ng/ml, more preferably 17.5±7.5 ng/ml, still more preferably 17.5±5.0 ng/ml, yet more preferably 17.5±2.5 ng/ml; or 20.0±10.0 ng/ml, more preferably 20.0±7.5 ng/ml, still more preferably 20.0±5.0 ng/ml, yet more preferably 20.0±2.5 ng/ml; or 25.0±10.0 ng/ml, more preferably 25.0±7.5 ng/ml, still more preferably 25.0±5.0 ng/ml, yet more preferably 25.0±2.5 ng/ml; or 30.0±10.0 ng/ml, more preferably 30.0±7.5 ng/ml, still more preferably 30.0±5.0 ng/ml, yet more preferably 30.0±2.5 ng/ml; or 35.0±10.0 ng/ml, more preferably 35.0±7.5 ng/ml, still more preferably 35.0±5.0 ng/ml, yet more preferably 35.0±2.5 ng/ml; or 40.0±10.0 ng/ml, more preferably 40.0±7.5 ng/ml, still more preferably 40.0±5.0 ng/ml, yet more preferably 40.0±2.5 ng/ml; or 45.0±10.0 ng/ml, more preferably 45.0±7.5 ng/ml, still more preferably 45.0±5.0 ng/ml, yet more preferably 45.0±2.5 ng/ml; or 50.0±10.0 ng/ml, more preferably 50.0±7.5 ng/ml, still more preferably 50.0±5.0 ng/ml, yet more preferably 50.0±2.5 ng/ml.

In further preferred embodiments, the dosage form according to the invention provides preferably under fasted conditions a mean value for tₘₐₓ within the range of 4.0±2.5 h; more preferably 4.0±2.0 h, still more preferably 4.0±1.5 h, yet more preferably 4.0±1.0 h; or 5.0±2.5 h; more preferably 5.0±2.0 h, still more preferably 5.0±1.5 h, yet more preferably 5.0±1.0 h; or 6.0±2.5 h; more preferably 6.0±2.0 h, still more preferably 6.0±1.5 h, yet more preferably 6.0±1.0 h; or 7.0±2.5 h; more preferably 7.0±2.0 h, still more preferably 7.0±1.5 h, yet more preferably 7.0±1.0 h.

Another aspect of the invention relates to a dosage form according to the invention as described above for use in the treatment of pain.

Preferably, the pain is moderate to severe. Preferably, the pain is acute pain or chronic pain. Preferably, the pain is nociceptive pain or neuropathic pain.

In a preferred embodiment, the dosage form according to the invention is for administration to a pediatric patient, preferably having an age of from 2 to 8 years, more preferably 2 to 6 years.

In another preferred embodiment, the dosage form according to the invention is for administration to a geriatric patient, preferably having an age of at least 70 years, more preferably at least 75 years.

In yet another preferred embodiment, the dosage form according to the invention is for administration to an adult, preferably having an age of at least 14 years, more preferably at least 18 years.

In still another preferred embodiment, the dosage form according to the invention is for administration to a patients with gastric or duodenal tubes.

Preferably, the dosage form according to the invention is for administration once daily, twice daily or thrice daily. Preferably, the dosage form according to the invention is for administration twice daily.

The dosage form according to the invention is an oral dosage form. Thus, the dosage form according to the invention is preferably for peroral administration, optionally after removing the coated particles from the remainder of the original dosage form, e.g. from the sprinkle capsule.

Particularly preferred embodiments A¹ to A²⁴ of the dosage form according to the invention are summarized in the tables here below, wherein all values are provided in wt. %, relative to the total weight of the coated particles:

| Ingredient [wt.-%] | A¹ | A² | A³ | A⁴ | A⁵ | A⁶ | A⁷ | A⁸ |
|---|---|---|---|---|---|---|---|---|
| Core - Inert carrier | 73±20 | 73±18 | 73±16 | 73±14 | 73±12 | 73±10 | 73±8 | 73±6 |
| Tapentadol component | 13±10 | 13±9 | 13±8 | 13±7 | 13±6 | 13±5 | 13±4 | 13±3 |
| Polymer component | 10±8 | 10±7 | 10±6 | 10±5 | 10±4 | 10±3 | 10±2 | 10±1 |
| Lubricant component | 3.0±2.5 | 3.0±2.2 | 3.0±1.9 | 3.0±1.6 | 3.0±1.3 | 3.0±1.0 | 3.0±0.7 | 3.0±0.4 |

| Ingredient [wt.-%] | A⁹ | A¹⁰ | A¹¹ | A¹² | A¹³ | A¹⁴ | A¹⁵ | A¹⁶ |
|---|---|---|---|---|---|---|---|---|
| Core - Inert carrier | 73±20 | 73±18 | 73±16 | 73±14 | 73±12 | 73±10 | 73±8 | 73±6 |
| Tapentadol HCl | 13±10 | 13±9 | 13±8 | 13±7 | 13±6 | 13±5 | 13±4 | 13±3 |
| Ethylcellulose | 10±8 | 10±7 | 10±6 | 10±5 | 10±4 | 10±3 | 10±2 | 10±1 |
| Magnesium stearate | 3.0±2.5 | 3.0±2.2 | 3.0±1.9 | 3.0±1.6 | 3.0±1.3 | 3.0±1.0 | 3.0±0.7 | 3.0±0.4 |

| Ingredient [wt.-%] | A¹⁷ | A¹⁸ | A¹⁹ | A²⁰ | A²¹ | A²² | A²³ | A²⁴ |
|---|---|---|---|---|---|---|---|---|
| Microcrystalline cellulose | 73±20 | 73±18 | 73±16 | 73±14 | 73±12 | 73±10 | 73±8 | 73±6 |
| Tapentadol HCl | 13±10 | 13±9 | 13±8 | 13±7 | 13±6 | 13±5 | 13±4 | 13±3 |
| Ethylcellulose | 10±8 | 10±7 | 10±6 | 10±5 | 10±4 | 10±3 | 10±2 | 10±1 |
| Magnesium stearate | 3.0±2.5 | 3.0±2.2 | 3.0±1.9 | 3.0±1.6 | 3.0±1.3 | 3.0±1.0 | 3.0±0.7 | 3.0±0.4 |

Another aspect of the disclosure, not encompassed by the wording of the claims, relates to a process for the manufacture of a pharmaceutical dosage form according to the invention as described above, the process comprising the step of (b) applying a controlled release coating material, which comprises a lubricant component, which comprises or essentially consists of magnesium stearate, and a polymer component, comprising one or more cellulose ethers, to a plurality of cores, which comprise a Tapentadol component.

Preferably, the controlled release coating material is applied in form of an ethanolic composition.

Preferably, the process according to the invention comprises the preceding step of
(a) applying a drug coat, which comprises the Tapentadol component, to a plurality of starter pellets thereby providing the plurality of cores.

Preferably, the drug coat is applied in form of an aqueous composition.

Preferably, in step (a) the drug coat and/or in step (b) the controlled release coat is applied by fluid-bed coating.

The following examples further illustrate the invention but are not to be construed as limiting its scope.

Tapentadol prolonged release granules were developed, considering that the used excipients have to be acceptable for pediatric use. Only excipients were selected which were sufficiently tested to be non-hazardous for children in the respective age group of 2 to 6 years.

### Comparative Example 1:

A comparative formulation development approach was based on a purely aqueous film-coating process. Therefore, pellets were produced via an extrusion/spheronization process and further processed by an aqueous solvent based prolonged release film-coating step.

The obtained granules demonstrated prolonged-release characteristics *in vitro* in aqueous dissolution media, but the dissolution profiles were not stable during storage, leading to accelerated drug release over time. Tapentadol HCl is a highly soluble drug substance which is known to alter the prolonged-release behavior of a formulation due to the migration into the film-coat in an aqueous environment.

### Comparative Example 2:

The manufacturing process was switched from aqueous to organic solvent based prolonged-release film coating. An initial drug layering was performed followed by a second organic solvent based coating step.

Ethanol 96% was used for the organic solvent based coating process. The solvent was removed during processing, and was therefore of no toxicological concern.

Different excipients e.g. cellulose acetate and ethylcellulose were investigated regarding their prolonged-release capability. Further, additives such as talcum were assessed as in the coating suspension to minimize agglomeration and electrostatic charging of the pellets during manufacturing.

All batches were evaluated based on their manufacturability, their dissolution and (short term) stability characteristics.

However, these formulations were not satisfactory in every respect. For example, the combination of ethylcellulose and talcum showed substantial disadvantages with respect to electrostatic charging during processing and an insufficient robustness.

### Example 1:

For clinical trials, three prototype formulations with different prolonged-release dissolution profiles were developed. This was achieved by applying different amounts of functional coating layer (all weight percent after drying).

Three different controlled release granules were manufactured as pluralities of coated particles. Each plurality of coated particles comprised a total dose of 29.12 mg Tapentadol hydrochloride (corresponding to 15.00 mg Tapentadol free base). The quantitative compositions of the three different formulations are provided in the table here below:

| Component | Function | Quantity per unit [mg] | | |
|---|---|---|---|---|
| | | 1-1 | 1-2 | 1-3 |
| Microcrystalline cellulose Ph. Eur. (Cellets^{®} 350) | starter pellet | 162.17 | 162.17 | 162.17 |
| Tapentadol HCl | active ingredient | 29.12 | 29.12 | 29.12 |
| Hypromellose Ph. Eur. | binder | 1.46 | 1.46 | 1.46 |
| Silicon dioxide Ph. Eur. | glidant | 1.46 | 1.46 | 1.46 |
| Ethylcellulose Ph. Eur. | polymer component | 22.40 | 29.87 | 37.34 |
| Magnesium stearate Ph. Eur. | lubricant component | 6.73 | 8.97 | 11.22 |
| Total | | 223.35 | 233.05 | 242.76 |

The polymer component and the lubricant component were applied as controlled release coating material. The relative weight contents of the three different formulations are provided in the table here below:

| Component | Arrangement | Percentage per unit [wt.-%] | | |
|---|---|---|---|---|
| | | 1-1 | 1-2 | 1-3 |
| Microcrystalline cellulose Ph. Eur. (Cellets^{®} 350) | starter pellet | 72.61 | 69.59 | 66.80 |
| Tapentadol HCl | drug coat | 13.04 | 12.50 | 12.00 |
| Hypromellose Ph. Eur. | | 0.65 | 0.63 | 0.60 |
| Silicon dioxide Ph. Eur. | | 0.65 | 0.63 | 0.60 |
| *Sub-total drug coat* | | *14.34* | *13. 76* | *13.20* |
| Ethylcellulose Ph. Eur. | controlled release coat | 10.03 | 12.82 | 15.38 |
| Magnesium stearate Ph. Eur. | | 3.01 | 3.85 | 4.62 |
| *Sub-Total controlled release coat* | | *13.04* | *16.67* | *20.00* |
| Total | | 100.00 | 100.00 | 100.00 |

The controlled release granules differed from one another essentially in the amounts of controlled release coating material.

Relative to the total weight of the controlled release granules, the granules of Example 1-1 contained about 13.0 wt.-% controlled release coating material, the granules of Example 1-2 contained about 16.7 wt.-% controlled release coating material, and the granules of Example 1-3 contained about 20.0 wt.-% controlled release coating material.

Relative to the partial weight of the starter pellet and the drug coat, the granules of Example 1-1 contained 15.0 wt.-% controlled release coating material, the granules of Example 1-2 contained 20.0 wt.-% controlled release coating material, and the granules of Example 1-3 contained 25.0 wt.-% controlled release coating material.

The manufacture involved two individual coating steps. In a first step, a drug layer was applied to starter pellets in an aqueous based coating step leading to Tapentadol immediate release granules. These Tapentadol immediate release granules were then further processed in a second, organic coating step to obtain Tapentadol prolonged release granules.

The manufacturing process involved only standard techniques of blending, fluid-bed coating and filling into glass vials.

Starter pellets of microcrystalline cellulose (28.390 kg) were coated with a composition comprising the following components in order to apply a drug coat:

| | |
|---|---|
| Tapentadol HCl | 5.098 kg |
| Hypromellose | 0.256 kg |
| Silicon dioxide | 0.256 kg |
| Purified water | 22.431 kg |

The thus obtained coated starter pellets (Tapentadol immediate release granules) were overcoated with a composition comprising the following components in order to apply a controlled release coat:

| | 1-1 | 1-2 | 1-3 |
|---|---|---|---|
| coated starter pellets | 4000.0 g | 4000.0 g | 4000.0 g |
| Ethylcellulose | 461.4 g | 615.2 g | 769.0 g |
| Magnesium stearate | 138.6 g | 184.8 g | 231.0 g |
| Ethanol 96% | 5400.0 g | 7200.0 g | 9000.0 g |
| Total | 4600.0 g | 4800.0 g | 5000.0 g |

The prolonged release granules of Examples 1-1 and 1-3 were tested for storage stability, for 8 months at 25 °C/60% r.h. as well as for 8 months at 40 °C/75% r.h. A shelf-life of at least 8 months was observed.

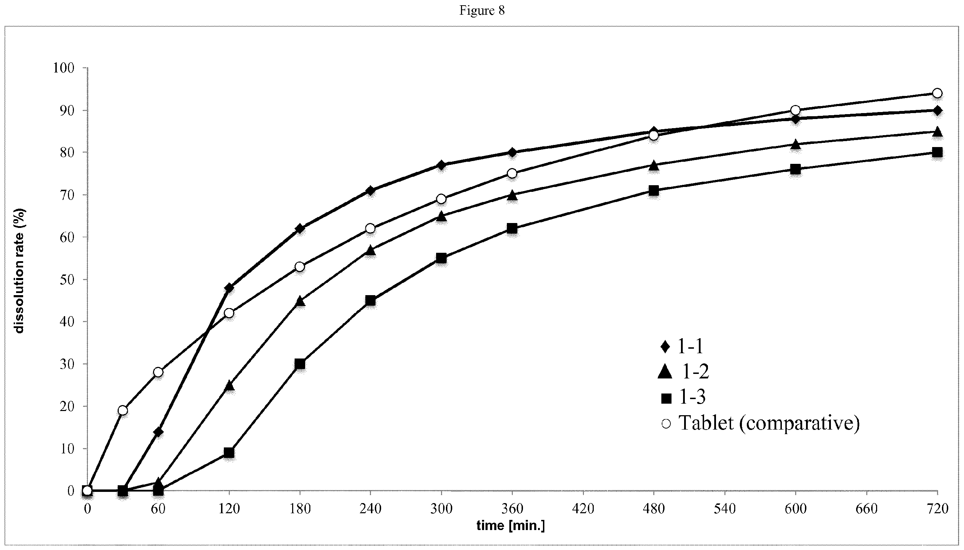

The *in vitro* release profiles are displayed in Figure 8 in comparison to a commercial tablet comprising the same amount of the Tapentadol component. The measurement was performed with the paddle method according to Ph. Eur. at 50 rpm in 900 ml phosphate buffer pH 6.8 and 37 °C.

The measured values for composition 1-1 are also provided in the table here below (mean, n=6):

| min | % released | | min | % released |
|---|---|---|---|---|
| 0 | 0.00 | | 390 | 78.35 |
| 30 | 0.06 | | 420 | 80.04 |
| 60 | 3.26 | | 450 | 81.50 |
| 90 | 19.76 | | 480 | 82.79 |
| 120 | 35.24 | | 510 | 83.93 |
| 150 | 45.80 | | 540 | 84.95 |
| 180 | 53.73 | | 570 | 85.89 |
| 210 | 59.78 | | 600 | 86.67 |
| 240 | 64.52 | | 630 | 87.43 |
| 270 | 68.39 | | 660 | 88.09 |
| 300 | 71.55 | | 690 | 88.71 |
| 330 | 74.17 | | 720 | 89.31 |
| 360 | 76.41 | | | |

Size and size distribution of the coated particles was investigated for several samples by sieve analysis. In the following table all values are provided as [wt.-%] where every line represents the measurement on one sample ("µ" = µm):

### Example 1-1:

| >315µ | >355µ | >400µ | >500µ | >560µ | >630µ | >710µ | >800µ | >900µ | >1000µ |
|---|---|---|---|---|---|---|---|---|---|
| 0 | 0.9 | 89.2 | 9.7 | 0.2 | 0.4 | 0.1 | 0.2 | 0 | 0 |
| 0 | 0.7 | 58.4 | 32.4 | 2.2 | 1.1 | 3.5 | 1.1 | 0.3 | 0.1 |
| 0 | 1.08 | 68.01 | 27.07 | 1.08 | 0.79 | 1.48 | 0.3 | 0.1 | 0 |
| 0 | 1.39 | 55.22 | 34.73 | 1.89 | 1.19 | 3.78 | 1.19 | 0.4 | 0.2 |
| 0.1 | 1.47 | 55.5 | 35.07 | 1.57 | 1.28 | 3.14 | 1.08 | 0.49 | 0.1 |
| 0.1 | 1.58 | 59.29 | 32.91 | 1.48 | 0.89 | 2.47 | 0.69 | 0.2 | 0.2 |
| 0.2 | 1.29 | 57.96 | 32.74 | 1.68 | 1.38 | 3.46 | 0.89 | 0.1 | 0 |
| 0.2 | 1.47 | 59.61 | 33.24 | 1.47 | 0.78 | 2.06 | 0.59 | 0.29 | 0.2 |
| 0.2 | 1.48 | 61.71 | 32.09 | 1.18 | 0.69 | 1.67 | 0.69 | 0.1 | 0.2 |
| 0.1 | 1.28 | 55.77 | 40.08 | 0.79 | 0.39 | 0.89 | 0.3 | 0.1 | 0.2 |
| 0 | 0.8 | 59.12 | 36.69 | 1 | 0.6 | 1.3 | 0.2 | 0.1 | 0.1 |

### Example 1-2:

| >315µ | >355µ | >400µ | >500µ | >560µ | >630µ | >710µ | >800µ | >900µ | >1000µ |
|---|---|---|---|---|---|---|---|---|---|
| 0.1 | 0.5 | 85.7 | 13.1 | 0.3 | 0.3 | 0.3 | 0 | 0 | 0 |
| 0 | 0.9 | 85.5 | 12.8 | 0.3 | 0.3 | 0.2 | 0 | 0 | 0 |

### Example 1-3:

| >315µ | >355µ | >400µ | >500µ | >560µ | >630µ | >710µ | >800µ | >900µ | >1000µ |
|---|---|---|---|---|---|---|---|---|---|
| 0.1 | 0.2 | 78.5 | 20.1 | 0.5 | 0.1 | 0.3 | 0.2 | 0 | 0 |
| 0 | 0.5 | 73.9 | 21.3 | 1 | 1 | 1.9 | 0.4 | 0 | 0 |
| 0.1 | 0.5 | 71.7 | 19.4 | 1.0 | 2.7 | 3.5 | 0.8 | 0.2 | 0.1 |

The average number of pellets was also determined thus allowing to provide an average weight of one coated particle (n=3):
125.6 mg of plurality coated particles = 1202 coated particles
125.6 mg of plurality coated particles = 1143 coated particles
126.3 mg of plurality coated particles = 1291 coated particles

Thus, an average of 125.83 mg of plurality coated particles corresponded to an average number of 1212 pellets. Accordingly, the average weight per particle was 103.82 µg.

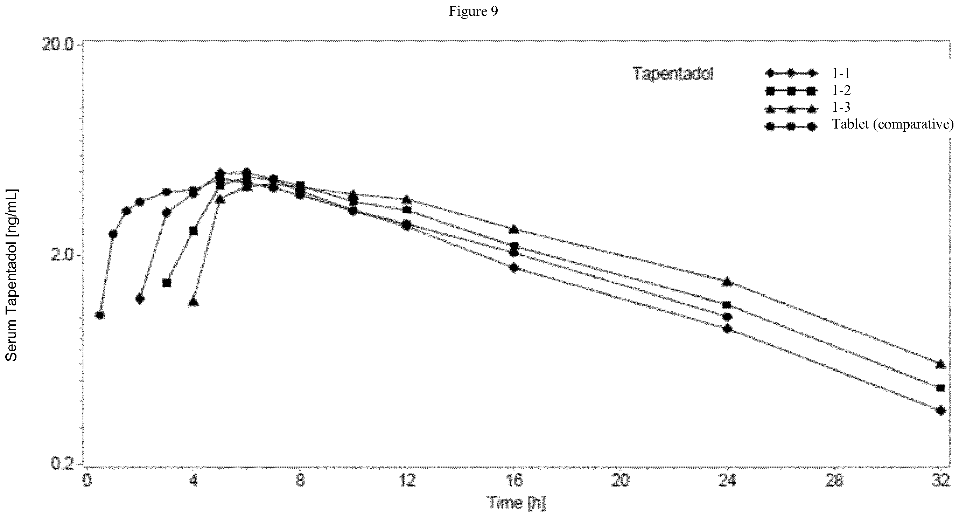

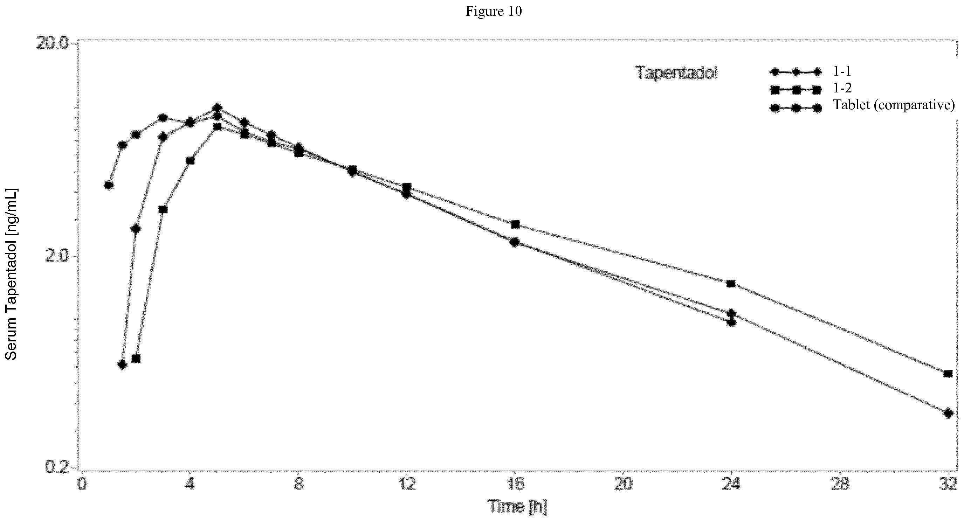

The pharmacokinetic parameters were determined in a clinical trial for the fasted status as well as for the fed status. The results are displayed in Figure 9 (fasted state) and Figure 10 (fed state) and are compiled in the table here below:

| | | Tablet (comparative) | | 1-1 | | 1-2 | | 1-3 |
|---|---|---|---|---|---|---|---|---|
| | | Fasted | Fed | Fasted | Fed | Fasted | Fed | Fasted |
| t_{lag} [h] | Mean ± SD (%CV) | 0.065 ± 0.172 (264%) | 0.197 ± 0.251 (128%) | 1.33 ± 0.287 (21.6%) | 1.07 ± 0.172 (16.2%) | 1.83 ± 0.243 (13.3%) | 1.59 ± 0.248 (15.6%) | 2.61 ± 0.499 (19.1%) |
| | Median (Range) | 0.000 (0.000 - 0.500) | 0.000 (0.000 - 0.533) | 1.50 (1.00 - 2.00) | 1.00 (1.00 - 1.50) | 2.00 (1.50 - 2.00) | 1.50 (1.00 - 2.03) | 3.00 (2.00 - 3.00) |
| tₘₐₓ [h] | Mean ± SD (%CV) | 5.24 ± 1.91 (36.4%) | 3.98 ± 1.59 (40.0%) | 5.61 ± 0.783 (13.9%) | 4.78 ± 0.672 (14.0%) | 6.61 ± 1.27 (19.2%) | 5.39 ± 0.840 (15.6%) | 7.30 ± 1.96 (26.9%) |
| | Median (Range) | 5.00 (1.50 - 10.0) | 4.00 (1.50 - 8.00) | 6.00 (4.00 -7.00) | 5.00 (3.00 - 6.00) | 7.00 (5.00 - 10.0) | 5.00 (4.00 - 8.00) | 7.00 (5.00 - 12.0) |
| Cₘₐₓ [ng/mL] | Mean ± SD (%CV) | 4.88 ± 1.74 (35.6%) | 10.0 ± 2.01 (20.1%) | 5.22 ± 1.83 (35.1%) | 10.2 ± 2.39 (23.4%) | 4.99 ± 1.51 (30.2%) | 8.59 ± 2.25 (26.2%) | 4.68 ± 1.65 (35.1%) |
| | Median (Range) | 4.66 (2.70 - 8.97) | 9.85 (7.01 - 14.8) | 4.88 (2.86 - 10.1) | 102 (6.88 - 14.9) | 4.53 (2.76 - 7.83) | 8.19 (4.89 - 12.8) | 4.47 (2.18 - 8.12) |
| tₗₐₛₜ [h] | Mean ± SD (%CV) | 29.6 ± 3.76 (12.7%) | 28.5 ± 4.72 (16.5%) | 31.3 ± 2.30 (7.36%) | 31.0 ± 2.75 (8.90%) | 31.3 ± 2.31 (7.36%) | 31.7 ± 1.66 (5.25%) | 31.7 ± 1.67 (5.27%) |
| | Median (Range) | 32.0 (24.0 - 32.0) | 32.0 (16.0 - 32.0) | 32.0 (24.0 - 32.0) | 32.0 (24.0 - 32.0) | 32.0 (24.0 - 32.0) | 32.0 (24.0 - 32.0) | 32.0 (24.0 - 32.0) |
| AUC₀₋ₜ [h* ng/mL] | Mean ± SD (%CV) | 67.7 ± 19.0 (28.0%) | 104 ± 25.3 (24.4%) | 61.0 ± 17.2 (28.1%) | 98.3 ± 20.9 (21.2%) | 64.3 ± 18.5 (28.7%) | 93.3 ± 21.9 (23.4%) | 67.9 ± 20.3 (29.9%) |
| | Median (Range) | 68.7 (37.6 - 104) | 102 (64.1 - 154) | 54.9 (35.4 - 107) | 92.9 (53.5 - 136) | 64.8 (28.4 - 115) | 89.8 (49.4 - 143) | 68.9 (30.8 - 108) |
| AUC [h* ng/mL] | Mean ± SD (%CV) | 73.5 ± 20.7 (28.2%) | 107 ± 25.2 (23.5%) | 65.1 ± 17.6 (27.1%) | 102 ± 21.0 (20.6%) | 69.8 ± 18.5 (26.6%) | 100 ± 24.0 (24.0%) | 76.1 ± 20.1 (26.4%) |
| | Median (Range) | 74.9 (43.3 - 118) | 105 (68.0 - 157) | 58.4 (41.4 - 115) | 95.9 (57.1 - 138) | 70.2 (35.6 - 120) | 92.7 (51.6 - 152) | 75.3 (42.3 - 120) |
| t_{1/2} [h] | Mean ± SD (%CV) | 6.95 ± 1.87 (26.9%) | 5.35 ± 1.02 (19.0%) | 6.99 ± 0.957 (13.7%) | 5.96 ± 1.04 (17.4%) | 7.33 ± 1.42 (19.4%) | 6.96 ± 1.68 (24.1%) | 8.27 ± 2.15 (26.1%) |
| | Median (Range) | 6.94 (3.42 - 11.5) | 5.64 (2.90 - 6.61) | 6.94 (5.32 - 9.48) | 5.80 (4.58 - 8.78) | 7.03 (5.48 - 11.1) | 6.77 (4.33 - 10.4) | 8.11 (4.98 - 15.1) |
| Cl/F [L/h] | Mean ± SD (%CV) | 368 ± 107 (29.2%) | 245 ± 55.0 (22.5%) | 408 ± 98.3 (24.1%) | 256 ± 57.5 (22.5%) | 383 ± 105 (27.5%) | 265 ± 68.2 (25.7%) | 351 ± 93.3 (26.6%) |
| | Median (Range) | 334 (212 - 578) | 237 (160 - 368) | 428 (217-604) | 261 (181 -438) | 356 (208 - 703) | 270 (164 -485) | 332 (208 - 591) |
| V_{z}/F [L] | Mean ± SD (%CV) | 3641 ± 1312 (36.0%) | 1887 ± 559 (29.6%) | 4156 ± 1356 (32.6%) | 2235 ± 857 (38.3%) | 4115 ± 1603 (39.0%) | 2613 ± 722 (27.6%) | 4287 ± 1886 (44.0%) |
| | Median (Range) | 3442 (1825 - 6155) | 1847 (1050 - 2868) | 3654 (2464 - 7822) | 2017 (1195 - 5440) | 3763 (1724 - 8729) | 2431 (1624 - 4121) | 3815 (1742 - 8992) |
| MRT [h] | Mean ± SD (%CV) | 13.1 ± 2.27 (17.3%) | 9.85 ± 1.25 (12.7%) | 13.5 ± 1.35 (10.1%) | 11.2 ± 0.910 (8.12%) | 15.2 ± 2.00 (13.1%) | 13.8 ± 2.15 (15.6%) | 17.6 ± 2.90 (16.4%) |
| | Median (Range) | 13.0 (7.32 - 18.7) | 102 (6.98 - 11.2) | 13.4 (11.8 - 16.6) | 11.2 (9.30 - 13.0) | 15.1 (11.5 - 19.8) | 13.7 (10.3 - 19.2) | 17.4 (13.6 - 26.9) |
| HVD [h] | Mean ± SD (%CV) | 13.4 ± 3.09 (23.1%) | 8.63 ± 1.57 (18.2%) | 9.89 ± 2.04 (20.7%) | 7.61 ± 1.89 (24.9%) | 11.2 ± 2.12 (18.9%) | 8.75 ± 2.68 (30.6%) | 13.9 ± 3.07 (22.1%) |
| | Median (Range) | 13.4 (8.57 - 19.6) | 8.38 (6.19 - 12.2) | 10.3 (3.45 - 13.2) | 7.23 (3.38 - 11.6) | 11.3 (7.34 - 14.4) | 7.88 (4.57 - 14.1) | 12.6 (10.4 - 20.6) |

The influence of food intake was determined for the coated pellets of Examples 1-1 and 1-2 and comped with the commercial tablet. The results are compiled in the following table:

| Parameter | Treatment | Fasted | Fed | |
|---|---|---|---|---|
| C ₘₐₓ [ng/mL] | Tablet (comparative) | 4.88 | 9.99 | |
| | 1-1 | 5.22 | 10.2 | |
| | 1-2 | 4.99 | 8.59 | |
| AUC₀₋ₜ [ng*li/mL] | Tablet (comparative) | 67.7 | 104 | |
| | 1-1 | 61.0 | 98.3 | |
| | 1-2 | 64.3 | 93.3 | |
| AUC[ng^{∗}h/mL | Tablet (comparative) | 73.5 | 107 | |
| | 1-1 | 65.1 | 102 | |
| | 1-2 | 69.8 | 99.8 | |

List of reference numerals
- 1 -: coated particle
- 2 -: inert core essentially comprising no Tapentadol component (e.g. nonpareil)
- 3 -: core comprising Tapentadol component
- 4 -: drug coat comprising Tapentadol component
- 5 -: controlled release coat comprising controlled release coating material
- 6 -: tamper resistant coat comprising tamper resistant coating material
- 6a -: inner layer of tamper resistant coat comprising alginate salt
- 6b -: outer layer of tamper resistant coat comprising acrylate polymer

### Example 2:

Two sets of controlled release granules were manufactured in accordance with Example 1-1. The only difference of the two sets was the particle size of the lubricant component (magnesium stearate) in the controlled release coating. All other parameters were identical such as content of Tapentadol, nature and content of further excipients, structure of granules and manufacturing conditions.

The particle size distribution of the magnesium stearate was measured according to different laser diffraction methods and the *in vitro* release of Tapentadol from the controlled release granules was determined in accordance with Example 1. The results are compiled in the table here below:

| [µm] | Example 2-1 | | | Example 2-2 | | | | |
|---|---|---|---|---|---|---|---|---|
| | not micronized | | | micronized | | | | |
| | dry, MasterSizer | Beckmann Coulter | | dry, MasterSizer | wet (Scharrsol D 80) Beckmann Coulter | | | |
| | | wet (H₂O, 1% Triton), | wet (Scharrsol D 80) | | #1 | #2 | #3 | #4 |
| Dx(10) | 1.310 | | | 0.276 | | | | |
| Dx(20) | 2.270 | | | 0.499 | | | | |
| Dx(30) | 3.330 | | | 0.896 | | | | |
| Dx(40) | 4.310 | | | 1.290 | | | | |
| Dx(50) | 5.300 | | | 1.650 | | | | |
| Dx(60) | 6.440 | | | 2.030 | | | | |
| Dx(70) | 7.920 | | | 2.460 | | | | |
| Dx(80) | 10.400 | | | 3.010 | | | | |
| Dx(90) | 18.000 | | | 3.860 | | | | |
| Dx(100) | 144.000 | | | 8.490 | | | | |
| D[3,2] | 2.560 | | | 0.772 | | | | |
| D[4,3] | 8.520 | | | 1.880 | | | | |
| mean | | 14.54 | 14.97 | | 1.596 | 1.684 | 1.720 | 1.765 |
| SD | | 13.65 | 12.84 | | 0.808 | 0.859 | 0.827 | 0.917 |
| median | | | 9.968 | | 1.456 | 1.539 | 1.605 | 1.614 |
| maximum | | 8.537 | 8.537 | | 1.748 | 1.919 | 1.919 | 2.107 |
| d₁₀ | | 3.075 | | | | | | |
| d₅₀ | | 9.610 | | | | | | |
| d₉₀ | | 35.73 | | | | | | |
| <10% | | | 3.137 | | 0.651 | 0.677 | 0.719 | 0.686 |
| <50% | | | 9.968 | | 1.456 | 1.539 | 1.605 | 1.614 |
| <75% | | | 22.01 | | 2.129 | 2.241 | 2.275 | 2.365 |
| <90% | | | 35.09 | | 2.773 | 2.917 | 2.901 | 3.090 |
| <95% | | | 41.92 | | 3.133 | 3.320 | 3.257 | 3.494 |
| <99% | | | 53.08 | | 3.741 | 3.990 | 3.841 | 4.199 |
| < 100% | | | 83.90 | | 5.111 | 5.610 | 5.611 | 6.159 |
| <2 µm | | | 3.59% | | 71.0% | 67.5% | 65.9% | 64.0% |
| <4 µm | | | 15.9% | | 99.6% | 99.0% | 99.4% | 98.2% |
| release after 60 min | 2 % (2-2 %) | | | | 35 % (34-37 %) | | | |
| release after 150 min | 44 % (43-44 %) | | | | 72 % (71-73 %) | | | |
| release after 720 min | 91 % (90-91 %) | | | | 97 % (96-99 %) | | | |

## Claims

1. An oral pharmaceutical dosage form comprising a plurality of coated particles, wherein said coated particles comprise a core which comprises a Tapentadol component, wherein under *in vitro* conditions the pharmaceutical dosage form provides controlled release of the Tapentadol component, and wherein the core is coated with a controlled release coating material, wherein the controlled release coating material comprises a lubricant component and a polymer component, wherein the lubricant component comprises or essentially consists of magnesium stearate, wherein the polymer component comprises one or more cellulose ethers, and wherein the relative weight ratio of the polymer component to the lubricant component is within the range of from 5.0:1.0 to 2.0: 1.0.

2. The dosage form according to claim 1, wherein the controlled release coating material essentially consists of the lubricant component and the polymer component.

3. The dosage form according to claim 1 or 2, wherein the polymer component comprises or essentially consists of a cellulose ether selected from the group consisting of ethylcellulose, hydroxyethylcellulose, propylcellulose, hydroxypropylcellulose, hydroxypropyl methylcellulose, and mixtures thereof; preferably wherein the cellulose ether is ethylcellulose.

4. The dosage form according to any of the preceding claims, wherein the lubricant component additionally comprises a fatty acid, a further metallic salt of a fatty acid, a fatty acid ester, an inorganic material, a polymeric lubricant, or a mixture thereof.

5. The dosage form according to claim 4, wherein
- the fatty acid is selected from stearic acid, myristic acid, palmitic acid, and mixtures thereof; and/or
- the further metallic salt of a fatty acid is selected from calcium stearate, zinc stearate, and mixtures thereof; and/or
- the fatty acid ester is selected from glyceride esters, preferably selected from glyceryl monostearate, glyceryl tribehenate, glyceryl dibehenate, and mixtures thereof; and sugar esters, preferably selected from sorbitan monostearate, sucrose monopalmitate, and mixtures thereof; and mixtures thereof; and/or
- the inorganic material is talc; and/or
- the polymeric lubricant is macrogol.

6. The dosage form according to any of the preceding claims, wherein the weight content of the controlled release coating material, relative to the total weight of the coated particles, is within the range of from 5.0 wt.-% to 21 wt.-%; preferably not more than 19 wt.-%; more preferably within the range of 13.0±4.0 wt.-%.

7. The dosage form according to any of the preceding claims, wherein the coated particles comprise a core which comprises a drug coat and a controlled release coat, wherein the drug coat comprises the Tapentadol component and wherein the controlled release coat comprises the controlled release coating material; preferably wherein the drug coat and the controlled release coat are in intimate contact with one another.

8. The dosage form according to any of the preceding claims, wherein the relative weight ratio of the polymer component to the lubricant component is within the range of from 5.0:1.0 to 2.1:1.0; preferably within the range of from 4.4:1.0 to 2.4:1.0; more preferably within the range of from 3.5:1.0 to 3.0:1.0.

9. The dosage form according to any of the preceding claims, wherein
- the weight content of the polymer component, relative to the total weight of the controlled release coating material, is within the range of 77.0±9.0 wt.-%; and/or
- the weight content of the lubricant component, relative to the total weight of the controlled release coating material, is within the range of 25±9.0 wt.-%.

10. The dosage form according to any of the preceding claims, which provides an *in vitro* release profile measured with the basket method according to Ph. Eur. at 100 rpm in 900 ml artificial gastric juice at pH 1.2 and 37 °C, such that
- after 60 min 15±10 wt.-%;
- after 120 min 50±30 wt.-%;
- after 240 min 70±20 wt.-%;
- after 360 min 80±15 wt.-%; and
- after 720 min 90±10 wt.-%;
of the Tapentadol component that was originally contained in the dosage form have been released.

11. The dosage form according to any of the preceding claims, wherein the particles are additionaly coated with a tamper resistant coating material providing resistance against ethanolic dose dumping.

12. The dosage form according to claim 11, wherein the tamper resistant coating material comprises a mixture of guar gum and ethylcellulose.

13. The dosage form according to claim 11, wherein the tamper resistant coating material comprises an inner layer and an outer layer, whereas the inner layer comprises an alginate salt and the outer layer comprises an acrylate polymer.

14. The dosage form according to any of the preceding claims for use in the treatment of pain.

15. The dosage form for use according to claim 14, wherein the dosage form is administered to a pediatric patient.

## Patentansprüche

1. Orale pharmazeutische Darreichungsform, umfassend eine Vielzahl überzogener Partikel, wobei die überzogenen Partikel einen Kern umfassen, der eine Tapentadol-Komponente umfasst, wobei die pharmazeutische Darreichungsform unter *In-vitro-*Bedingungen eine kontrollierte Freisetzung der Tapentadol-Komponente ermöglicht und wobei der Kern mit einem Überzugsmaterial mit kontrollierter Freisetzung überzogen ist, wobei das Überzugsmaterial mit kontrollierter Freisetzung eine Gleitmittelkomponente und eine Polymerkomponente umfasst, wobei die Gleitmittelkomponente Magnesiumstearat umfasst oder im Wesentlichen daraus besteht, wobei die Polymerkomponente einen oder mehrere Celluloseether umfasst und wobei das relative Gewichtsverhältnis der Polymerkomponente zu der Gleitmittelkomponente im Bereich von 5,0:1,0 bis 2,0:1,0 liegt.

2. Darreichungsform nach Anspruch 1, wobei das Überzugsmaterial mit kontrollierter Freisetzung im Wesentlichen aus der Gleitmittelkomponente und der Polymerkomponente besteht.

3. Darreichungsform nach Anspruch 1 oder 2, wobei die Polymerkomponente einen Celluloseether umfasst oder im Wesentlichen daraus besteht, ausgewählt aus der Gruppe bestehend aus Ethylcellulose, Hydroxyethylcellulose, Propylcellulose, Hydroxypropylcellulose, Hydroxypropylmethylcellulose und Mischungen davon; wobei der Celluloseether vorzugsweise Ethylcellulose ist.

4. Darreichungsform nach einem der vorhergehenden Ansprüche, wobei die Gleitmittelkomponente zusätzlich eine Fettsäure, ein weiteres Metallsalz einer Fettsäure, einen Fettsäureester, ein anorganisches Material, ein polymeres Gleitmittel oder eine Mischung davon umfasst.

5. Darreichungsform nach Anspruch 4, wobei
die Fettsäure aus Stearinsäure, Myristinsäure, Palmitinsäure und Mischungen davon ausgewählt ist; und/oder
das weitere Metallsalz einer Fettsäure aus Calciumstearat, Zinkstearat und Mischungen davon ausgewählt ist; und/oder
der Fettsäureester aus Glyceridestern ausgewählt ist, vorzugsweise ausgewählt aus Glycerylmonostearat, Glyceryltribehenat, Glyceryldibehenat und Mischungen davon; und Zuckerester, vorzugsweise ausgewählt aus Sorbitanmonostearat, Saccharosemonopalmitat und Mischungen davon; und Mischungen davon; und/oder
das anorganische Material Talk ist; und/oder
das polymere Gleitmittel Macrogol ist.

6. Darreichungsform nach einem der vorhergehenden Ansprüche, wobei der Gewichtsanteil des Überzugsmaterials mit kontrollierter Freisetzung, bezogen auf das Gesamtgewicht der überzogenen Partikel, im Bereich von 5,0 Gew.-% bis 21 Gew.-% liegt; vorzugsweise nicht mehr als 19 Gew.-%; mehr bevorzugt im Bereich von 13,0 ± 4,0 Gew.-%.

7. Darreichungsform nach einem der vorhergehenden Ansprüche, wobei die überzogenen Partikel einen Kern umfassen, der einen Arzneimittelüberzug und einen Überzug mit kontrollierter Freisetzung umfasst, wobei der Arzneimittelüberzug die Tapentadol-Komponente umfasst und wobei der Überzug mit kontrollierter Freisetzung das Überzugsmaterial mit kontrollierter Freisetzung umfasst; wobei der Arzneimittelüberzug und der Überzug mit kontrollierter Freisetzung vorzugsweise in engem Kontakt miteinander stehen.

8. Darreichungsform nach einem der vorhergehenden Ansprüche, wobei das relative Gewichtsverhältnis der Polymerkomponente zu der Gleitmittelkomponente im Bereich von 5,0:1,0 bis 2,1:1,0 liegt; vorzugsweise im Bereich von 4,4:1,0 bis 2,4:1,0; mehr bevorzugt im Bereich von 3,5:1,0 bis 3,0:1,0.

9. Darreichungsform nach einem der vorhergehenden Ansprüche, wobei
der Gewichtsanteil der Polymerkomponente, bezogen auf das Gesamtgewicht des Überzugsmaterials mit kontrollierter Freisetzung, im Bereich von 77,0 ± 9,0 Gew.-% liegt; und/oder
der Gewichtsanteil der Gleitmittelkomponente, bezogen auf das Gesamtgewicht des Überzugsmaterials mit kontrollierter Freisetzung, im Bereich von 25 ± 9,0 Gew.-% liegt.

10. Darreichungsform nach einem der vorhergehenden Ansprüche, die ein In-vitro-Freisetzungsprofil bereitstellt, gemessen mit der Drehkörbchenmethode gemäß Ph. EUR. bei 100 U/min in 900 ml künstlichem Magensaft bei pH 1,2 und 37 °C, so dass
nach 60 min 15110 Gew.-%;
nach 120 min 50130 Gew.-%;
nach 240 min 70120 Gew.-%;
nach 360 min 80±15 Gew.-%; und
nach 720 min 90110 Gew.-%;
der ursprünglich in der Darreichungsform enthaltenen Tapentadol-Komponente freigesetzt wird.

11. Darreichungsform nach einem der vorhergehenden Ansprüche, wobei die Partikel zusätzlich mit einem manipulationssicheren Überzugsmaterial überzogen sind, das einem Alkohol-Dose-Dumping entgegenwirkt.

12. Darreichungsform nach Anspruch 11, wobei das manipulationssichere Überzugsmaterial eine Mischung aus Guarkernmehl und Ethylcellulose umfasst.

13. Darreichungsform nach Anspruch 11, wobei das manipulationssichere Überzugsmaterial eine Innenschicht und eine Außenschicht umfasst, wobei die Innenschicht ein Alginatsalz und die Außenschicht ein Acrylatpolymer umfasst.

14. Darreichungsform nach einem der vorhergehenden Ansprüche zur Verwendung bei der Schmerzbehandlung.

15. Darreichungsform zur Verwendung nach Anspruch 14, wobei die Darreichungsform einem pädiatrischen Patienten verabreicht wird.

## Revendications

1. Forme posologique pharmaceutique orale comprenant une pluralité de particules enrobées, dans laquelle lesdites particules enrobées comprennent un noyau qui comprend un composant Tapentadol, dans laquelle, dans des conditions *in vitro,* la forme posologique pharmaceutique fournit une libération contrôlée du composant Tapentadol, et dans laquelle le noyau est enrobé avec un matériau d'enrobage à libération contrôlée, dans laquelle le matériau d'enrobage à libération contrôlée comprend un composant lubrifiant et un composant polymère, dans laquelle le composant lubrifiant comprend ou est essentiellement constitué par le stéarate de magnésium, dans laquelle le composant polymère comprend un ou plusieurs éthers de cellulose, et dans laquelle le rapport pondéral relatif entre le composant polymère et le composant lubrifiant est compris dans la plage allant de 5,0:1,0 à 2,0:1,0.

2. Forme posologique selon la revendication 1, dans laquelle le matériau d'enrobage à libération contrôlée est essentiellement constitué par le composant lubrifiant et le composant polymère.

3. Forme posologique selon la revendication 1 ou 2, dans laquelle le composant polymère comprend ou est essentiellement constitué par un éther de cellulose choisi dans le groupe constitué par l'éthylcellulose, l'hydroxyéthylcellulose, la propylcellulose, l'hydroxypropylcellulose, l'hydroxypropylméthylcellulose et des mélanges de ceux-ci ; de préférence dans laquelle l'éther de cellulose est l'éthylcellulose.

4. Forme posologique selon l'une quelconque des revendications précédentes, dans laquelle le composant lubrifiant comprend en outre un acide gras, un autre sel métallique d'un acide gras, un ester d'acide gras, une matière inorganique, un lubrifiant polymère ou un mélange de ceux-ci.

5. Forme posologique selon la revendication 4, dans laquelle
- l'acide gras est choisi parmi l'acide stéarique, l'acide myristique, l'acide palmitique et des mélanges de ceux-ci ; et/ou
- l'autre sel métallique d'un acide gras est choisi parmi le stéarate de calcium, le stéarate de zinc et des mélanges de ceux-ci ; et/ou
- l'ester d'acide gras est choisi parmi les esters de glycéride, de préférence choisi parmi le monostéarate de glycéryle, le tribéhénate de glycéryle, le dibéhénate de glycéryle et des mélanges de ceux-ci ; et les esters de sucre, de préférence choisis parmi le monostéarate de sorbitan, le monopalmitate de saccharose et des mélanges de ceux-ci ; et des mélanges de ceux-ci ; et/ou
- la matière inorganique est du talc ; et/ou
- le lubrifiant polymère est le macrogol.

6. Forme posologique selon l'une quelconque des revendications précédentes, dans laquelle la teneur en poids du matériau d'enrobage à libération contrôlée, par rapport au poids total des particules enrobées, est comprise dans la plage allant de 5,0 % en poids à 21 % en poids ; de préférence ne dépasse pas 19 % en poids ; est comprise de manière davantage préférée dans la plage de 13,0 ± 4,0 % en poids.

7. Forme posologique selon l'une quelconque des revendications précédentes, dans laquelle les particules enrobées comprennent un noyau qui comprend un enrobage de médicament et un enrobage à libération contrôlée, dans laquelle l'enrobage de médicament comprend le composant Tapentadol et dans laquelle l'enrobage à libération contrôlée comprend le matériau d'enrobage à libération contrôlée ; de préférence dans laquelle l'enrobage de médicament et l'enrobage à libération contrôlée sont en contact étroit l'un avec l'autre.

8. Forme posologique selon l'une quelconque des revendications précédentes, dans laquelle le rapport pondéral relatif entre le composant polymère et le composant lubrifiant est compris dans la plage allant de 5,0:1,0 à 2,1:1,0 ; de préférence dans la plage allant de 4,4:1,0 à 2,4:1,0 ; de manière davantage préférée dans la plage allant de 3,5:1,0 à 3,0:1,0.

9. Forme posologique selon l'une quelconque des revendications précédentes, dans laquelle
- la teneur en poids du composant polymère, par rapport au poids total du matériau d'enrobage à libération contrôlée, est comprise dans la plage de 77,0 ± 9,0 % en poids ; et/ou
- la teneur en poids du composant lubrifiant, par rapport au poids total du matériau d'enrobage à libération contrôlée, est comprise dans la plage de 25 ± 9,0 % en poids.

10. Forme posologique selon l'une quelconque des revendications précédentes, qui fournit un profil de libération *in vitro* mesuré avec la méthode du panier selon Ph. EUR. à 100 tr/min dans 900 ml de suc gastrique artificiel à pH 1,2 et 37 °C, de sorte que
- après 60 min 15 ± 10 % en poids ;
- après 120 min 50 ± 30 % en poids ;
- après 240 min 70 ± 20 % en poids ;
- après 360 min 80 ± 15 % en poids ; et
- après 720 min 90 ± 10 % en poids ;
du composant Tapentadol contenu à l'origine dans la forme posologique ont été libérés.

11. Forme posologique selon l'une quelconque des revendications précédentes, dans laquelle les particules sont en outre enrobées d'un matériau d'enrobage inviolable fournissant une résistance contre le déversement de dose éthanolique.

12. Forme posologique selon la revendication 11, dans laquelle le matériau d'enrobage inviolable comprend un mélange de gomme de guar et d'éthylcellulose.

13. Forme posologique selon la revendication 11, dans laquelle le matériau de revêtement inviolable comprend une couche interne et une couche externe, tandis que la couche interne comprend un sel d'alginate et la couche externe comprend un polymère d'acrylate.

14. Forme posologique selon l'une quelconque des revendications précédentes destinée à être utilisée dans le traitement de la douleur.

15. Forme posologique destinée à être utilisée selon la revendication 14, dans laquelle la forme posologique est administrée à un patient pédiatrique.
